# EUROPEAN PATENT APPLICATION

(11) **EP 2 105 447 A1**
(43) Date of publication of application: **30.09.2009**
(21) Application number: 07860273.7
(22) Date of filing: 19.12.2007
(51) Int. Cl.: C07K 16/18, G01N 33/53

(54) **AVIAN-DERIVED ANTIBODY CAPABLE OF BINDING SPECIFICALLY TO HUMAN HMGB1, IMMUNOLOGICAL DETERMINATION METHOD FOR HUMAN HMGB1, AND IMMUNOLOGICAL DETERMINATION REAGENT FOR HUMAN HMGB1**

(30) Priority: 20.12.2006 JP 2006357368
(71) Applicant: Shino-Test Corporation, Tokyo101-8410 (JP)
(72) Inventor: YAMADA, Shingo, Sagamihara-shi Kanagawa 229-0011 (JP); YAKABE, Keiko, Sagamihara-shi Kanagawa 229-0011 (JP)
(74) Representative: Denison, Christopher Marcus
(86) International application number: PCT/JP2007/075051
(87) International publication number: WO 2008/075788

(57) **Abstract**

An object of the present invention is to provide an anti-human HMGB1 antibody having high capability of binding to human high mobility group box protein-1 (HMGB1) with a high probability. According to the present invention, a highly sensitive immunoassay method and immunoassay reagent for human HMGB1 in a sample using the anti-human HMGB1 antibody are provided. Specifically, provided herein are: an avian-derived anti-human HMGB1 antibody specifically binding to the amino acid sequence shown by the following formula (I): Lys Pro Asp Ala Ala Lys Lys Gly Val Val Lys Ala Glu Lys Ser (I) of human HMGB1; and an immunoassay method and an immunoassay reagent for human HMGB1 in a sample, which are **characterized by** using the antibody.

## Description

### Technical Field

The present invention relates to an avian-derived antibody specifically binding to human HMGB1, which can be a marker for diseases such as septicemia, an immunoassay method for human HMGB1 in a sample that uses the antibody, an immunoassay reagent for human HMGB1 in a sample that contains the antibody, a method for obtaining an avian-derived anti-human HMGB1 antibody, and a method for improving productivity for obtaining the anti-human HMGB1 antibody.

The present invention is useful in life science fields such as laboratory tests, clinical pathology, immunology, and medicine and chemical fields such as analytical chemistry, for example.

### Background Art

HMGBs (High Mobility Group Box Proteins) were previously referred to as HMGs (High Mobility Group Proteins). They were discovered in large amounts in 1964 as nonhistone proteins contained in chromatin structure. The proteins are universally contained in all higher animals and plants, exhibiting an extremely high degree of conservation of primary structures among species.

It is also known that the proteins are richly present not only in nuclei, but also in cytoplasm.

The physiological action of the proteins has not yet been clearly elucidated. However, since HMGBs loosen the double helix structure upon binding to DNA, HMGBs are thought to function as extremely wide-ranging transcriptional accelerators and nucleosome relaxing factors that alter the higher order structure of DNA to result in an optimum structure upon transcription so as to enhance transcriptional activity.

There are several types of HMGB.

Examples of HMGB include HMGB1 (high mobility group box protein 1), HMGB2 (high mobility group box protein 2), HMGB3 (high mobility group box protein 3), HMGB8 (high mobility group box protein 8), HMGB17 (high mobility group box protein 17), HMGB I (high mobility group box protein I), HMGB Y (high mobility group box protein Y), HMGB I(Y) (high mobility group box protein I(Y)), and HMGB I-C (high mobility group box protein I-C).

In addition, the present inventors have analyzed amino acid sequence homology using "GENETYX" genetic information processing software (SOFTWARE DEVELOPMENT). As a result, cattle HMGB1 has been found to have the homology of 98.6% with human HMGB1 and swine HMGB1 has been found to have the homology of 99.1% with human HMGB1.

Furthermore, similarly, human HMGB2 has homology of 81.2% with human HMGB1, human HMGB2 has homology of 72.3% with cattle HMGB2, and human HMGB2 has homology of 79.4% with swine HMGB2.

In 1999, Wang et al., performed for the first time the quantitative measurement of HMGB1 in serum (in blood) by the Western blot method using a polyclonal antibody prepared using HMGB 1 itself as an immunogen.

As a result, Wang et al., demonstrated that HMGB1 can serve as a marker for septicemia.

It was thus demonstrated that discrimination between septicemia patients that will survive and septicemia patients that will not survive is possible via precise measurement of HMGB1 in blood (see H. Wang et al., SCIENCE, Vol. 285, No. 9, pp. 248-251, issued 1999).

In addition, it has previously been shown by Parkkinen et al., and Lepp et al., that antibodies to be used for measurement of HMGB1; that is, antibodies binding to HMGB1, can be prepared (see J. Parkkinen et al., The Journal of Biological Chemistry, Vol. 268, No. 26, pp. 19726-19738, issued 1993; and W. A. Lepp et al., Journal of Immunoassay, Vol. 10, No. 4, pp. 449-465, issued 1989).

With the use of such antibodies, Lepp et al., have said that solid-phase enzyme immunoassay is possible for HMGB1.

Also, Cabart et al., have described a method for preparing human HMGB1 and human HMGB2 (see P. Cabart et al., Cell Biochemistry and Function 13, pp. 125-133, issued 1995).

Also, documents have been published describing that HMGB1 is also induced upon inflammation, which may cause secretion of various cytokines in large amounts (see Andersson, U et al., J. Exp. Med., Vol. 192, pp. 565-570, issued 2000; Scaffidl et al., Nature, Vol. 418, pp. 191-195, issued 2002; and Park et al., The Journal of Biological Chemistry, Vol. 279, No. 27, issued 2004).

Accordingly, accurate and precise quantitative measurement of HMGB1 is clearly more useful than qualitative confirmation by which the presence or the absence of HMGB 1 in blood is simply confirmed.

In addition, the present inventors have previously developed an antibody that binds to human HMGB1 but does not bind to human HMGB2, and an immunoassay reagent and an immunoassay method for human HMGB1, by which accurate measurement values containing no errors can be obtained without the measurement of HMGB2 (see JP Patent Publication (Kokai) No. 2003-96099 A).

### Disclosure of the Invention

### Object to Be Attained by the Invention

As described above, accurate and precise quantitative measurement of human HMGB1 in a sample has been required. However, it has never been easy to obtain an anti-human HMGB1 antibody that can be used for such accurate and precise quantitative measurement of human HMGB1; that is, an antibody having high capability of binding (titer) to human HMGB1.

The reasons are as described below.

To prepare an antibody that specifically binds to a substance to be measured, in general, animals that are easy to rear (e.g., rabbits, goats, sheep, mice, or rats) are immunized with the whole or a part of the substance to be measured or a conjugate of such substance and a carrier bound thereto, via injection or the like.

Also, when an animal is immunized with a substance to be measured or a conjugate of a substance to be measured and a carrier, in general, various measures have been taken to induce and obtain an antibody having high capability of binding to a substance to be measured, such that an adjuvant is mixed with a substance to be measured and the like.

Meanwhile, the present inventors have attempted to obtain an anti-human HMGB1 antibody specifically binding to human HMGB 1 by immunizing mammals such as rabbits, mice, or cattle with human HMGB1 or a part thereof via injection, but were only able to obtain extremely small amounts of such antibody having high capability of binding to human HMGB1.

For example, when 4 rabbits were each immunized with human HMGB1 via injection, an anti-human HMGB1 antibody having high capability of binding to human HMGB1 could not be obtained. All antibodies obtained from these 4 rabbits were low-titer antibodies having low capability of binding.

Also, for example, when 10 rabbits were each immunized with a part of human HMGB1 via injection, an anti-human HMGB1 antibody having high capability of binding to human HMGB1 was obtained from only one out of the 10 rabbits. Moreover, all antibodies obtained from the remaining 9 rabbits were low-titer antibodies having low binding capacity.

Therefore, the present inventors have confirmed that: generally when mammals to be used as immune animals for obtainment of antibodies, such as rabbits, mice, or cattle, are immunized with the whole or a part of human HMGB1, most of the products are low-titer antibodies having low capability of binding to human HMGB1; and the probability of obtaining high-titer antibodies having high capability of binding to human HMGB1 is extremely low.

Hence, as a result of intensive studies to obtain with a high probability a high-titer antibody having high capability of binding to human HMGB1, the present inventors have discovered that the object can be achieved by using birds as immune animals and a peptide containing a specific amino acid sequence as an immunogen. Thus, the present inventors have completed the present invention.

### Means for Attaining the Object

The present inventors have continuously examined the following issues: the reason why most of antibodies obtained by immunizing animals such as rabbits, mice, or cattle with the whole or a part of human HMGB1 are low-titer antibodies having low capability of binding to human HMGB1; and furthermore, the reason why the probability of obtaining high-titer antibodies having high capability of binding to human HMGB1 is extremely low.

As a result, the present inventors have discovered that when animals such as rabbits, mice, or cattle are immunized with human HMGB1 or a part thereof via injection, HMGB1 of the animals is generated *in vivo* in the animals.

This is inferred as follows. Human HMGB1 is foreign matter for animals that are immunized therewith via injection, so that introduction of such foreign matter into their bodies will result in great stress for them. In response to the stress, HMGB1 of the animals is generated *in vivo* as a reactant in the animals.

Generally, the amino acid sequence (primary structure) of HMGB1 of animals (e.g., rabbits, goats, sheep, mice, and rats) that are used for antibody production and antibody obtainment differs from that of human HMGB1 by only 2 to 3 amino acid residues.

Accordingly, "HMGB1 of these animals, which is generated *in vivo* as a reactant in these animals as a result of immunization of these animals with human HMGB1 via injection" has extremely high homology with human HMGB1. Hence, it binds *in vivo* to "an anti-human HMGB1 antibody produced *in vivo* in these animals as a result of immunization of these animals with human HMGB1 via injection."

Therefore, when such anti-human HMGB1 antibodies produced *in vivo* in these animals have bound to HMGB1 produced *in vivo* in these animals (or most such anti-human HMGB1 antibodies have bound to the same), the anti-human HMGB1 antibodies cannot further bind to human HMGB1 (or few of them can bind). Hence, it is inferred that such anti-human HMGB1 antibodies are low-titer antibodies having low capability of binding to human HMGB1, and that the probability of obtaining high-titer antibodies having high capability of binding to human HMGB1 is extremely low.

As described above, the present inventors have discovered for the first time that when animals such as rabbits, mice, or cattle are immunized with human HMGB1 or a part thereof via injection, HMGB1 of the animals is generated *in vivo* in these animals. Hence, the present inventors have conceived for the first time that since the thus generated HMGB1 of the animals binds to produced anti-human HMGB1 antibodies *in vivo* in these animals, the thus obtained anti-human HMGB1 antibodies are low-titer antibodies and the probability of obtaining high-titer antibodies is extremely low.

Based on the findings of the present inventors, the present inventors have paid special attention to birds because the amino acid sequence (primary structure) of avian HMGB1 has low homology with the same of human HMGB1. The present inventors have discovered for the first time that high-titer antibodies having high capability of binding to human HMGB1 can be obtained with high probability through the use of birds as immune animals and a peptide as an immunogen containing a part of the amino acid sequence of human HMGB1 that has low homology with the amino acid sequence of avian HMGB1.

Specifically, the present invention comprises the following (1) to (23).
(1) An avian-derived anti-human HMGB1 antibody, comprising an avian-derived antibody specifically binding to the amino acid sequence shown by the following formula (I): Lys Pro Asp Ala Ala Lys Lys Gly Val Val Lys Ala Glu Lys Ser (I) of human HMGB1.
(2) The avian-derived anti-human HMGB1 antibody according to (1) above, which is obtained by immunizing birds with:
   a peptide comprising the amino acid sequence shown by the following formula (I): Lys Pro Asp Ala Ala Lys Lys Gly Val Val Lys Ala Glu Lys Ser (I) of human HMGB1;
   a peptide comprising an amino acid sequence obtained by subjecting the amino acid sequence shown by formula (I) to deletion, substitution, insertion, addition, or modification of one to several amino acid residues; or
   a conjugate of the peptide and a carrier
   as an immunogen.
(3) The avian-derived anti-human HMGB1 antibody according to (1) or (2) above, wherein the avian-derived anti-human HMGB1 antibody has high capability of binding to human HMGB1 and the productivity thereof is high.
(4) The avian-derived anti-human HMGB1 antibody according to any one of (1) to (3) above, wherein the birds are chickens.
(5) An immunoassay method for measuring human HMGB1 in a sample using an antigen-antibody reaction with an antibody specifically binding to the human HMGB1, wherein an avian-derived anti-human HMGB1 antibody that is an avian-derived antibody specifically binding to the amino acid sequence shown by the following formula (I): Lys Pro Asp Ala Ala Lys Lys Gly Val Val Lys Ala Glu Lys Ser (I) of human HMGB1 is used.
(6) The immunoassay method for human HMGB1 in a sample according to (5) above, wherein the avian-derived anti-human HMGB1 antibody is obtained by immunizing birds with:
   a peptide comprising the amino acid sequence shown by the following formula (I): Lys Pro Asp Ala Ala Lys Lys Gly Val Val Lys Ala Glu Lys Ser (I) of human HMGB1;
   a peptide comprising an amino acid sequence obtained by subjecting the amino acid sequence shown by formula (I) to deletion, substitution, insertion, addition, or modification of one to several amino acid residues; or
   a conjugate of the peptide and a carrier
   as an immunogen.
(7) The immunoassay method for human HMGB1 in a sample according to (5) or (6) above, wherein the avian-derived anti-human HMGB1 antibody has high capability of binding to human HMGB1 and the productivity thereof is high.
(8) The immunoassay method for human HMGB1 in a sample according to any one of (5) to (7) above, which is a highly sensitive immunoassay method capable of measuring human HMGB1 with high sensitivity.
(9) The immunoassay method for human HMGB1 in a sample according to any one of (5) to (8) above, wherein the birds are chickens.
(10) An immunoassay reagent for measuring human HMGB1 in a sample using an antigen-antibody reaction with an antibody specifically binding to the human HMGB1, containing an avian-derived anti-human HMGB1 antibody that is an avian-derived antibody specifically binding to the amino acid sequence shown by the following formula (I): Lys Pro Asp Ala Ala Lys Lys Gly Val Val Lys Ala Glu Lys Ser (I) of human HMGB1.
(11) The immunoassay reagent for human HMGB1 in sample according to (10) above, wherein the avian-derived anti-human HMGB1 antibody is obtained by immunizing birds with:
   a peptide comprising the amino acid sequence shown by the following formula (I): Lys Pro Asp Ala Ala Lys Lys Gly Val Val Lys Ala Glu Lys Ser (I) of human HMGB1;
   a peptide comprising an amino acid sequence obtained by subjecting the amino acid sequence shown by formula (I) to deletion, substitution, insertion, addition, or modification of one to several amino acid residues; or
   a conjugate of the peptide and a carrier
   as an immunogen.
(12) The immunoassay reagent for human HMGB1 in a sample according to (10) or (11) above, wherein the avian-derived anti-human HMGB1 antibody has high capability of binding to human HMGB1 and the productivity thereof is high.
(13) The immunoassay reagent for human HMGB1 in a sample according to any one of (10) to (12) above, which is a highly sensitive immunoassay reagent capable of measuring human HMGB1 with high sensitivity.
(14) The immunoassay reagent for human HMGB1 in a sample according to any one of (10) to (13) above, wherein the productivity of the immunoassay reagent is improved.
(15) The immunoassay reagent for human HMGB1 in a sample according to any one of (10) to (14) above, wherein the birds are chickens.
(16) A method for obtaining an avian-derived anti-human HMGB1 antibody, comprising immunizing birds with:
   a peptide comprising the amino acid sequence shown by the following formula (I): Lys Pro Asp Ala Ala Lys Lys Gly Val Val Lys Ala Glu Lys Ser (I) of human HMGB1;
   a peptide comprising an amino acid sequence obtained by subjecting the amino acid sequence shown by formula (I) to deletion, substitution, insertion, addition, or modification of one to several amino acid residues; or
   a conjugate of the peptide and a carrier
   as an immunogen.
(17) The method for obtaining an avian-derived anti-human HMGB1 antibody according to (16) above, wherein the avian-derived anti-human HMGB1 antibody is capable of specifically binding to the amino acid sequence shown by the following formula (I): Lys Pro Asp Ala Ala Lys Lys Gly Val Val Lys Ala Glu Lys Ser (I) of human HMGB1.
(18) The method for obtaining an avian-derived anti-human HMGB1 antibody according to (16) or (17) above, wherein the avian-derived anti-human HMGB1 antibody has high capability of binding to human HMGB1 and the productivity thereof is high.
(19) The method for obtaining an avian-derived anti-human HMGB1 antibody according to any one of (16) to (18) above, wherein the birds are chickens.
(20) A method for improving the productivity for obtaining an anti-human HMGB1 antibody, comprising immunizing birds with:
   a peptide comprising the amino acid sequence shown by the following formula (I): Lys Pro Asp Ala Ala Lys Lys Gly Val Val Lys Ala Glu Lys Ser (I) of human HMGB1;
   a peptide comprising an amino acid sequence obtained by subjecting the amino acid sequence shown by formula (I) to deletion, substitution, insertion, addition, or modification of one to several amino acid residues; or
   a conjugate of the peptide and a carrier
   as an immunogen.
(21) The method for improving the productivity for obtaining an anti-human HMGB1 antibody according to (20) above, wherein the anti-human HMGB1 antibody is capable of specifically binding to the amino acid sequence shown by the following formula (I): Lys Pro Asp Ala Ala Lys Lys Gly Val Val Lys Ala Glu Lys Ser (I) of human HMGB1.
(22) The method for improving the productivity for obtaining an anti-human HMGB1 antibody according to (20) or (21) above, wherein the anti-human HMGB1 antibody has high capability of binding to human HMGB1.
(23) The method for improving the productivity for obtaining an anti-human HMGB1 antibody according to any one of (20) to (22) above, wherein the birds are chickens.

### Effects of the Invention

The avian-derived anti-human HMGB1 antibody of the present invention is a high-titer antibody having high capability of binding to human HMGB1.

Also, the productivity of the avian-derived anti-human HMGB1 antibody of the present invention is high, such that a high-titer antibody having high capability of binding to human HMGB1 can be obtained with high probability.

The immunoassay method for human HMGB1 in a sample of the present invention is a highly sensitive immunoassay method, by which even trace amounts of human HMGB1 contained in a sample can be measured accurately.

The immunoassay reagent for human HMGB1 in a sample of the present invention is a highly sensitive immunoassay reagent capable of accurately measuring even trace amounts of human HMGB1 contained in a sample.

Also, the immunoassay reagent for human HMGB1 in a sample of the present invention is less likely to lead to production of rejected products (defective products) with low sensitivity for measurement and can be produced and obtained with good production yields and improved productivity.

The method for obtaining the avian-derived anti-human HMGB1 antibody of the present invention is a method by which a high-titer anti-human HMGB1 antibody having high capability of binding to human HMGB1 can be obtained.

Also, the method for obtaining the avian-derived anti-human HMGB1 antibody of the present invention is a productive method by which a high-titer anti-human HMGB1 antibody having high capability of binding to human HMGB1 can be obtained with high probability.

The method for improving the productivity for obtaining the anti-human HMGB1 antibody of the present invention allows the productivity for obtaining an anti-human HMGB1 antibody to be improved, by which a high-titer anti-human HMGB1 antibody having high capability of binding to human HMGB1 can be obtained with high probability.

### Brief Description of the Drawings

Fig. 1 shows the results of the Western blot method by which the reactivity of the avian-derived anti-human HMGB1 antibody of the present invention to human HMGB1 and human HMGB2 was confirmed.
Fig. 2 shows a calibration curve produced by measuring samples containing human HMGB1 with the use of the immunoassay reagent and the immunoassay method for human HMGB1 in a sample of the present invention.

### Best Modes for Carrying out the Invention

### [I] Avian-derived anti-human HMGB1 antibody

### (1) Antibody of the present invention

The "avian-derived anti-human HMGB1 antibody" of the present invention is an avian-derived antibody specifically binding to the amino acid sequence shown by the following formula (I): Lys Pro Asp Ala Ala Lys Lys Gly Val Val Lys Ala Glu Lys Ser (I) of human HMGB1.

The "avian-derived anti-human HMGB1 antibody" of the present invention is from birds and may be any such antibody capable of specifically binding to the amino acid sequence shown by the following formula (I): Lys Pro Asp Ala Ala Lys Lys Gly Val Val Lys Ala Glu Lys Ser (I) of human HMGB1.

The "avian-derived anti-human HMGB1 antibody" of the present invention may be any antibody as long as it is from birds.

Examples of such birds include chickens, quails, pheasants, ostriches, and ducks.

As such birds, birds of the family *Phasianidae* are preferred and chickens are particularly preferred.

In addition, the "avian-derived anti-human HMGB1 antibody" of the present invention may be any of a polyclonal antibody, antiserum containing a polyclonal antibody, a monoclonal antibody, or a fragment (e.g., Fab, F(ab')₂, or Fab') of such antibody, for example.

### (2) Immunogen

To obtain the "avian-derived anti-human HMGB1 antibody" of the present invention, an immunogen to be used for immunization of birds is as described below.

Examples of an immunogen for obtaining the "avian-derived anti-human HMGB1 antibody" of the present invention include a peptide comprising the amino acid sequence shown by the following formula (I): Lys Pro Asp Ala Ala Lys Lys Gly Val Val Lys Ala Glu Lys Ser (I) of human HMGB1, a peptide comprising an amino acid sequence obtained by subjecting the amino acid sequence shown by formula (I) to deletion, substitution, insertion, addition, or modification of one to several amino acid residues, and a conjugate of the peptide and a carrier.

In addition, the number of the above "several" amino acid residues to be deleted, substituted, inserted, added, or modified generally ranges from 1 to 4, preferably ranges from 1 to 3, further preferably ranges from 1 to 2, and is particularly preferably 1.

As the immunogen for obtaining the "avian-derived anti-human HMGB1 antibody" of the present invention, a peptide comprising the amino acid sequence shown by the following formula (I): Lys Pro Asp Ala Ala Lys Lys Gly Val Val Lys Ala Glu Lys Ser (I) or a conjugate of the peptide comprising the amino acid sequence shown by formula (I) and a carrier is preferred. Particularly, a conjugate of the peptide comprising the amino acid sequence shown by the above formula (I) and a carrier is preferred.

When birds are immunized with a peptide comprising the above amino acid sequence shown by formula (I), a peptide comprising an amino acid sequence obtained by subjecting the amino acid sequence shown by formula (I) to deletion, substitution, insertion, addition, or modification of one to several amino acid residues, or a conjugate of the peptide and a carrier as an immunogen, the thus obtained "avian-derived anti-human HMGB1 antibody" is capable of specifically binding to the above amino acid sequence shown by formula (I).

In this case, the above amino acid sequence shown by formula (I) corresponds to a part of the amino acid sequence of human HMGB1 having low homology with the amino acid sequence of avian HMGB1. Hence, the "avian-derived anti-human HMGB1 antibody" obtained by immunizing birds with the above immunogen has the following two characteristics.
(a) The avian-derived anti-human HMGB1 antibody is capable of specifically binding to human HMGB1; and
(b) The avian-derived anti-human HMGB1 antibody is unable to bind to (or bind to HMGB1 with difficulty) HMGB1 that is generated *in vivo* in the above birds as a result of immunization with the above immunogen. Thus, no avian-derived anti-human HMGB1 antibody binds to HMGB1 generated *in vivo* in the above birds (or the number of the avian-derived anti-human HMGB1 antibody binding to such HMGB1 is low). Therefore, the avian-derived anti-human HMGB1 antibody is a high-titer antibody having high capability of binding to human HMGB1 and can be obtained with high probability.

In addition, the above amino acid sequence shown by formula (I) also corresponds to a part of the amino acid sequence of human HMGB1 having low homology with the amino acid sequence of human HMGB2. Accordingly, when a peptide comprising the above amino acid sequence shown by formula (I), a peptide comprising an amino acid sequence obtained by subjecting the amino acid sequence shown by formula (I) to deletion, substitution, insertion, addition, or modification of one to several amino acid residues, or a conjugate of the peptide and a carrier is used as an immunogen, the thus obtained "avian-derived anti-human HMGB1 antibody" is also characterized by specifically binding to HMGB1, but not binding to HMGB2.

Therefore, also in this regard, the "avian-derived anti-human HMGB1 antibody" obtained using as an immunogen a peptide comprising the above amino acid sequence shown by formula (I), a peptide comprising an amino acid sequence obtained by subjecting the amino acid sequence shown by formula (I) to deletion, substitution, insertion, addition, or modification of one to several amino acid residues, or a conjugate of the peptide and a carrier is useful because of its specificity to human HMGB1.

### (3) Binding specificity and productivity

The "avian-derived anti-human HMGB1 antibody" of the present invention is a high-titer antibody having high capability of binding to human HMGB1, as described above.

Furthermore, the "avian-derived anti-human HMGB1 antibody" of the present invention is characterized by its high productivity thereof such that a high-titer antibody having high capability of binding to human HMGB1 can be obtained with high probability.

### (4) Method for obtaining an immunogen for the antibody of the present invention

To obtain the "avian-derived anti-human HMGB1 antibody" of the present invention, a method for obtaining an immunogen for immunization of birds is as described below.

Examples of the above immunogens include a peptide comprising the amino acid sequence shown by the following formula (I): Lys Pro Asp Ala Ala Lys Lys Gly Val Val Lys Ala Glu Lys Ser (I) of human HMGB1, a peptide comprising an amino acid sequence obtained by subjecting the amino acid sequence shown by formula (I) to deletion, substitution, insertion, addition, or modification of one to several (generally 1 to 4, preferably 1 to 3, further preferably 1 to 2, and particularly preferably 1 amino acid residue) amino acid residues, and a conjugate of the peptide and a carrier. They can be obtained via extraction, purification, or the like by a known method or the like from body fluids, cells, tissues, organs, or the like of a human or a non-human mammal (e.g., a pig, cattle, a rabbit, a goat, sheep, a mouse, or a rat) having an HMGB1 amino acid sequence that has high homology with that of human HMGB1.

Also, the above immunogens can be synthesized by a method for peptide synthesis, such as a liquid phase method or a solid phase method. Furthermore, an automatic peptide synthesizer can also be used herein.

For example, the above immunogens can be synthesized according to methods described in The Japanese Biochemical Society (Ed.,), "Laboratory Course in Biochemistry 1 (Seikagaku Jikken Koza 1) Protein Chemistry IV, TOKYO KAGAKU DOJIN CO., LTD, 1975; Izumiya et al., "Basics and Experiments for Peptide Synthesis," Maruzen, 1985; or The Japanese Biochemical Society (Ed.,), "Laboratory Course in Biochemistry 2, Part 2 (Zoku Seikagaku Jikken Koza 2) Protein Chemistry Vol. 2," TOKYO KAGAKU DOJIN CO., LTD, 1987; or the like.

Furthermore, the above immunogens may be prepared by genetic engineering techniques from DNA or RNA having a nucleic acid or nucleotide sequence corresponding thereto, with reference to The Japanese Biochemical Society (Ed.,), "Laboratory Course in Biochemistry 1 Part 2 (Zoku Seikagaku Jikken Koza 1) Genetic Research Technique I (Idenshi Kenkyu Ho I)," TOKYO KAGAKU DOJIN CO., LTD, 1986; The Japanese Biochemical Society (Ed.,), "Laboratory Course in Biochemistry 1, Part 2 (Zoku Seikagaku Jikken Koza 1) Genetic Resesarch Techniques II (Idenshi Kenkyu Ho II," TOKYO KAGAKU DOJIN CO., LTD, 1986; The Japanese Biochemical Society (Ed.,), "Laboratory Course in Biochemistry 1 Part 2 (Zoku Seikagaku Jikken Koza 1) Genetic Research Techniques III (Idenshi Kenkyu Ho III," TOKYO KAGAKU DOJIN CO., LTD, 1987, or the like.

For example, a gene corresponding to the above amino acid sequence shown by formula (I) of human HMGB1 is cloned and then the thus obtained gene is incorporated into an expression vector such as a plasmid.

Next, the expression vector is introduced into a host cell such as *Escherichia coli*. The thus obtained transformant is cultured so that a peptide comprising the above amino acid sequence shown by formula (I) can be expressed.

In addition, examples of a method for cloning the nucleotide sequence of a gene include a PCR method, a recombinant PCR method, a ligation method, and a linker ligation method.

Meanwhile, when an immunogen is a low-molecular-weight substance, in general, an animal or the like is immunized with an immunogen to which a carrier has been bound. However, it has been reported that a specific antibody is produced using a peptide of 5 amino acids (the number of amino acids is 5) as an immunogen (Kiyama et al., "The Pharmaceutical Society of Japan, The 112th Annual Meeting Lecture Summaries 3," p. 122, issued 1992). Hence, the use of a carrier is not essential.

In addition, when a conjugate of a peptide comprising the above amino acid sequence shown by formula (I) and a carrier or a conjugate of a peptide comprising an amino acid sequence obtained by subjecting the amino acid sequence shown by formula (I) to deletion, substitution, insertion, addition, or modification of one to several amino acid residues and a carrier is used as an immunogen, examples of carriers that can be used herein include known carriers, such as keyhole limpet hemocyanin (KLH), bovine serum albumin (BSA), human serum albumin (HSA), chicken serum albumin, poly-L-lysine, polyalanyl lysine, dipalmityl lysine, tetanus toxoid, and polysaccharides.

Examples of a method for binding the above peptide to a carrier that can be used herein, include known binding methods such as a glutaraldehyde method, a 1-ethyl-3-(3-dimethylaminopropyl)carbodiimide method, a maleimidobenzoyl-N-hydroxysuccinimide ester method, a bisdiazotized benzidine method, and a N-succimidyl-3-(2-pyridyldithio)propionate method.

Furthermore, a carrier such as a nitrocellulose particle, polyvinylpyrrolidone, or a liposome to which the above peptide has been adsorbed can also be used as an immunogen in the present invention.

### (5) Method for obtaining the avian-derived anti-human HMGB1 antibody (polyclonal antibody) of the present invention

### (i) Obtainment of polyclonal antibody from serum

Regarding the "avian-derived anti-human HMGB1 antibody" of the present invention, a polyclonal antibody or antiserum can be obtained by the following procedures from the serum of birds immunized with an immunogen.

First, birds such as chickens are immunized with the above immunogen.

The amount of the above immunogen to be used for immunization is determined depending on the types of birds to be immunized, sites for immunization injection, and the like. In the case of chickens, 10 µg to 1 mg of the above immunogen is preferably injected per immunization injection into each chicken.

In addition, the above immunogen is preferably used for immunization injection after it has been added to and mixed with an adjuvant.

Examples of an adjuvant that can be used herein include known adjuvants such as Freund's complete adjuvant, Freund's incomplete adjuvant, an aluminum hydroxide adjuvant, and *Bordetella pertussis* adjuvant.

Immunization injection may be performed subcutaneously, intravenously, intraperitoneally, or to a site such as dorsal part.

After primary immunization, booster injections of the above immunogen are performed at intervals of 2 to 3 weeks subcutaneously, intravenously, intraperitoneally, or to a site such as dorsal part.

Also in this case, the above immunogen is preferably used for a booster injection after it has been added to and mixed with an adjuvant.

After primary immunization, serum antibody titers of immunized birds are measured repeatedly by ELISA or the like. When the antibody titers reach a plateau, exsanguination is performed and then serum is separated. Thus, antiserum containing the antibody of the present invention is obtained.

The antiserum is subjected to antibody purification that is performed by one of or a combination of a salting-out method using ammonium sulfate, sodium sulfate, or the like, ion exchange chromatography, gel filtration, affinity chromatography, and the like, so that polyclonal antibodies are obtained.

### (ii) Obtainment of polyclonal antibody from egg

Regarding the "avian-derived anti-human HMGB1 antibody" of the present invention, a polyclonal antibody can be obtained by the following procedures from eggs of birds immunized with an immunogen.

First, female birds such as hens are immunized with the above immunogen.

The amount of the above immunogen to be used for immunization is determined depending on the types of birds to be immunized, sites for immunization injection, and the like. In the case of chickens, 10 µg to 1 mg of the above immunogen is preferably injected per immunization injection into each hen.

In addition, the above immunogen is preferably used for an immunization injection after it has been added to and mixed with an adjuvant.

Examples of an adjuvant that can be used herein include known adjuvants such as Freund's complete adjuvant, Freund's incomplete adjuvant, an aluminum hydroxide adjuvant, and *Bordetella pertussis* adjuvant.

Immunization injection may be carried out subcutaneously, intravenously, intraperitoneally, or to a site such as dorsal part.

After primary immunization, booster injections of the above immunogen are performed at intervals of 2 to 3 weeks subcutaneously, intravenously, intraperitoneally, or to a site such as dorsal part.

Also in this case, the above immunogen is preferably used for a booster injection after it has been added to and mixed with an adjuvant.

After primary immunization, antibody titers in eggs laid by immunized birds are measured repeatedly by ELISA or the like. When the antibody titers reach a plateau, yolks are obtained from eggs laid after this timing.

The yolks are subjected to antibody purification that is performed by one of or a combination of methods such as a salting-out method using ammonium sulfate, sodium sulfate, or the like, ion exchange chromatography, gel filtration, and affinity chromatography, so that polyclonal antibodies are obtained.

### (iii) Absorption and removal of antibody from carrier

When birds are immunized with a conjugate of the above peptide and a carrier as an immunogen, an antibody corresponding to the carrier (that is, an antibody binding to the carrier) is present among the thus obtained antiserum or polyclonal antibodies. Hence, absorption and removal of an antibody corresponding to such carrier are preferably performed.

Examples of a method that can be used for such removal include a method that involves adding a carrier into a solution of the thus obtained polyclonal antibody or antiserum and then removing the thus generated aggregates and a method that involves immobilizing a carrier on insolubilized solid phase and then removing the carrier by affinity chromatography.

As described in (i), (ii), and (iii) above, a polyclonal antibody that is the "avian-derived anti-human HMGB1 antibody" of the present invention; that is, an avian-derived polyclonal antibody specifically binding to human HMGB1, can be isolated and obtained.

### (6) Method for obtaining the avian-derived anti-human HMGB1 antibody (monoclonal antibody) of the present invention

Regarding the "avian-derived anti-human HMGB1 antibody" of the present invention, a monoclonal antibody can be obtained by the following procedures.

Monoclonal antibodies can be obtained using hybridomas prepared by the cell fusion method of Koehler et al., (G. Koehler et al., Nature, Vol. 256, pp. 495-497, issued 1975) or antibody-producing cells such as tumorigenic cells obtained using viruses such as Epstein-Barr virus.

Monoclonal antibody preparation according to the cell fusion method can be performed by the following procedures.

First, birds such as chickens are immunized with the above immunogen.

The amount of the above immunogen to be used for immunization is determined depending on the types of birds to be immunized, sites for immunization injection, and the like. In the case of chickens, 10 µg to 1 mg of the above immunogen is preferably injected per immunization injection into each chicken.

In addition, the above immunogen is preferably used for an immunization injection after it has been added to and mixed with an adjuvant.

Examples of an adjuvant that can be used herein include known adjuvants such as Freund's complete adjuvant, Freund's incomplete adjuvant, an aluminum hydroxide adjuvant, and *Bordetella pertussis* adjuvant.

Immunization injection may be performed subcutaneously, intravenously, intraperitoneally, or to a site such as dorsal part.

After primary immunization, booster injections of the above immunogen are performed at intervals of 1 to 2 weeks subcutaneously, intravenously, intraperitoneally, or to a site such as dorsal part.

The number of booster injections generally ranges from 2 to 6 times. Also in this case, the above immunogen is preferably used for a booster injection after it has been added to and mixed with an adjuvant.

After primary immunization, antibody titers in the sera of or in eggs laid by immunized birds are measured repeatedly by ELISA or the like. When the antibody titers reach a plateau, the above immunogen is dissolved in normal saline (0.9% sodium chloride aqueous solution) and then injected intravenously or intraperitoneally for final immunization.

On days 3 to 5 after the final immunization, splenocytes, lymph node cells, peripheral lymphocytes, or the like of immunized birds, which are capable of producing antibodies, are obtained.

Cells capable of producing antibodies (e.g., splenocytes, lymph node cells, or peripheral lymphocytes) obtained from such immunized birds are fused to myeloma cells or B cells of birds, mammals, or the like. As myeloma cells, cells of a cell line deficient in an enzyme (e.g., hypoxanthine·guanine·phosphoribosyl·transferase (HGPRT) or thymidine kinase (TK)) are preferred. Examples of such cell line that can be used herein include BALB/c mouse-derived HGPRT-deficient cell lines, such as the P3-X63-Ag8 cell line (ATCC TIB9), the P3-X63-Ag8-U1 cell line (Research source bank for cancer research (JCRB) 9085), the P3-NS1-1-Ag4-1 cell line (JCRB 0009), the P3-X63-Ag8·653 cell line (JCRB 0028), and the SP2/O-Ag-14 cell line (JCRB 0029).

Cell fusion can be performed using a fusion accelerator such as various-molecular-weight polyethylene glycol (PEG), liposomes, Sendai virus (HVJ), and the like or performed via an electric fusion method or the like.

When myeloma cells are cells of an HGPRT-deficient cell line or a TK-deficient cell line, only fusion cells (hybridomas) of cells capable of producing antibodies and myeloma cells can be selectively cultured and proliferated using selection medium (HAT medium) containing hypoxanthine·aminopterin·thymidine.

The thus obtained culture supernatants of the above hybridomas are measured by an immunoassay method (e.g., ELISA or the Western blot method) using the above immunogen, human HMGB1, or the like. Thus, hybridomas that produce antibodies binding to human HMGB1 or the like can be selected.

The above culture supernatants of the above hybridomas are measured by an immunoassay method (e.g., ELISA or the Western blot method) using human HMGB2 or the like. Thus, hybridomas that produce antibodies not binding to human HMGB2 or the like can be selected.

The two types of hybridoma selection method and a known cloning method such as limiting dilution are performed in combination, so that a cell line producing: a monoclonal antibody that is the "avian-derived anti-human HMGB1 antibody" of the present invention and specifically an avian-derived monoclonal antibody specifically binding to human HMGB1 can be isolated and obtained.

The monoclonal antibody-producing cell line is cultured in an appropriate medium and then a monoclonal antibody that is the "avian-derived anti-human HMGB1 antibody" of the present invention can be obtained from the culture supernatants. As a medium, a serum free medium, a low concentration serum medium, or the like may be used. In this case, a DMEM medium, an RPMI1640 medium, an ASF medium 103, and the like can be preferably used since they facilitate antibody purification.

The thus obtained monoclonal antibodies are subjected to one of or a combination of a salting-out method using ammonium sulfate, sodium sulfate, or the like, ion exchange chromatography, gel filtration, and affinity chromatography, for example, so that purified monoclonal antibodies that are "avian-derived anti-human HMGB1 antibodies" of the present invention can be obtained.

### [II] Immunoassay method for human HMGB1 in sample

### (1) General statement

The immunoassay method (hereinafter, it may also be referred to as "the immunoassay method of the present invention" or "the measurement method of the present invention") for human HMGB1 in a sample of the present invention is an immunoassay method for measuring human HMGB1 in a sample using an antigen-antibody reaction with an antibody specifically binding to the human HMGB1. This immunoassay method is characterized by using "the avian-derived anti-human HMGB1 antibody ("avian-derived anti-human HMGB1 antibody") comprising an avian-derived antibody that specifically binds to the amino acid sequence shown by the following formula (I): Lys Pro Asp Ala Ala Lys Lys Gly Val Val Lys Ala Glu Lys Ser (I) of human HMGB1."

Particularly, the immunoassay method for human HMGB1 in a sample is an immunoassay method characterized by using the above "avian-derived anti-human HMGB1 antibody" as an antibody specifically binding to human HMGB1 that is a substance to be measured.

The immunoassay method of the present invention is a highly sensitive immunoassay method by which even trace amounts of human HMGB1 contained in a sample can be measured accurately using the above "avian-derived anti-human HMGB1 antibody"; that is, a high-titer antibody having high capability of binding to human HMGB1.

The "avian-derived anti-human HMGB1 antibody" can be used without any particular restrictions, as long as it is such an antibody, by which the above effects can be obtained.

In addition, in the immunoassay method using two or more of antibodies specifically binding to human HMGB1, at least one of the two or more antibodies is the above "avian-derived anti-human HMGB1 antibody."

Also, the other antibody may be any antibody, as long as it is an "antibody specifically binding to human HMGB1."

In addition, the two or more antibodies may also be the above "avian-derived anti-human HMGB1 antibodies." Moreover, all antibodies to be used herein may be the above "avian-derived anti-human HMGB1 antibodies."

For example, when a sandwich method is employed, such as the enzyme immunoassay (ELISA), fluorescence immunoassay, or luminescence immunoassay method, either or both of an antibody labeled with an enzyme or the like and an immobilized antibody are the above "avian-derived anti-human HMGB1 antibodies." Alternatively, both antibodies may be the above "avian-derived anti-human HMGB1 antibodies."

Regarding the "avian-derived anti-human HMGB1 antibody," not only 1 type thereof, but also a plural number of types thereof may also be used simultaneously.

The above "avian-derived anti-human HMGB1 antibody" may be any antibody as long as it is an avian-derived antibody.

Examples of birds to be used herein include chickens, quails, pheasants, ostriches, and ducks. Birds of the family *Phasianidae* are preferred and chickens are particularly preferred.

In addition, details concerning the "avian-derived anti-human HMGB1 antibody" are as described in the above section, "[I] Avian-derived anti-human HMGB1 antibody," and the like.

### (2) Immunoassay method

The immunoassay method of the present invention is an immunoassay method for measuring human HMGB1 in a sample using an antigen-antibody reaction of human HMGB1 with an antibody specifically binding to the human HMGB1. The measurement principle of this method is not particularly limited and desired effects are exerted by the method, as long as the above "avian-derived anti-human HMGB1 antibody" is used.

Examples of the immunoassay method include enzyme-linked immunosorbent assay or enzyme immunoassay (ELISA and EIA), fluorescence immunoassay (FIA), radioimmunoassay (RIA), luminescence immunoassay (LIA), an enzyme antibody method, a fluorescent antibody technique, an immunochromatographic method, immunonephelometry, latex turbidimetry, latex agglutination assay, a hemagglutination method, a particle agglutination method, a measurement method as described in JP Patent Publication (Kokai) No. 9-229936 A (1997), JP Patent Publication (Kokai) No. 10-132819 A (1998), or the like, in which a substance specifically binding to a substance to be measured (analyte) is immobilized and a carrier having a surface coated with such substance and particles to which a substance specifically binding to a substance to be measured (analyte) have been immobilized are used, and ELSA (Enzyme-linked Ligandsorbent Assay) as shown by Dahlbeack et al., (Thromb. Haemost., Vol. 79, pp. 767-772, issued 1998; WO98/23963).

Furthermore, regarding the above immunoassay methods, the immunoassay method of the present invention can be applied to any techniques such as a sandwich method, a competitive method, or a homogeneous method.

Also, measurement to be performed by the immunoassay method of the present invention may be performed using a manual method or using an apparatus such as an analyzer.

### (3) Sample

Examples of samples to be used in the immunoassay method of the present invention include body fluids such as human blood, serum, blood plasma, urine, seminal fluids, spinal fluids, saliva, sweat, tears, ascites, and amniotic fluids; feces; blood vessels or organs such as liver; tissues; and cells; or extracts obtained from feces, organs, tissues, cells, or the like, as long as they are biological samples or the like that can contain human HMGB1.

### (4) Immunoassay method using labeled antibody

When the immunoassay method of the present invention is performed by enzyme immunoassay, fluorescence immunoassay, radioimmunoassay, luminescence immunoassay, or the like, using a labeled antibody (or a labeled antigen) prepared by binding a labeling substance to an antibody (or an antigen), and an immobilized antibody (or an immobilized antigen) prepared by immobilizing an antibody (or an antigen) onto a solid phase carrier, the immunoassay method of the present invention can be performed by a sandwich method, a competitive method, or the like and is preferably performed by the sandwich method.

When the immunoassay method of the present invention is performed by the above sandwich method, either a labeled antibody or an immobilized antibody is the above "avian-derived anti-human HMGB1 antibody." Also, both labeled antibodies and immobilized antibodies may be the above "avian-derived anti-human HMGB1 antibodies."

Examples of solid phase carriers that are used for immobilized antibodies (or immobilized antigens) to be used in the above immunoassay method include solid phase carries in the form of microcapsules, beads, microplates (microtiter plates), test tubes, sticks, test strips, and the like, which are made of material such as polystyrene, polycarbonate, polyvinyl toluene, polypropylene, polyethylene, polyvinyl chloride, nylon, polymethacrylate, polyacrylamide, latex, liposome, gelatin, agarose, cellulose, sepharose, glass, ceramics, metal, or magnetic material.

An immobilized antibody (or an immobilized antigen) can be prepared according to a known method (e.g., physical adsorption, chemical binding, or a combination thereof) by causing adsorption or binding between an antibody such as the above "avian-derived anti-human HMGB1 antibody" and a solid phase carrier.

When physical adsorption is employed, such immobilized antibody can be prepared according to a known method by mixing and contacting an antibody (or an antigen) with a solid phase carrier in a solution such as a buffer, or contacting an antibody (or an antigen) dissolved in a buffer or the like with a solid phase carrier, for example.

Also, when chemical binding is employed, such immobilized antibody can be prepared according to a known method as described in the Japanese Society of Laboratory Medicine (Ed.), "Clinical Pathology, Extra Edition, No. 53, Immunoassay for Laboratory Tests -Techniques and Applications-," The Clinical Pathology Press, issued 1983; The Japanese Biochemical Society (Ed.), "New Biochemistry Experimental Lectures 1 (Shin Seikagaku Jikken Koza 1) Protein IV," TOKYO KAGAKU DOJIN CO., LTD, issued 1991, or the like, by mixing and contacting an antibody (or an antigen) and a solid phase carrier with a bifunctional crosslinking reagent such as glutaraldehyde, carbodiimide, imide ester, maleimide, or the like, so as to react with amino groups, carboxyl groups, thiol groups, aldehyde groups, hydroxyl groups, or the like of each of the antibody (or the antigen) and the solid phase carrier, for example.

Also, if there is a need to perform treatment for suppressing non-specific reactions, natural aggregation of a solid phase carrier, or the like, the surface or inner wall surface of a solid phase carrier onto which an antibody (or an antigen) has been immobilized may be treated by a known method that involves contacting and coating the same with a protein (e.g., bovine serum albumin (BSA), human serum albumin (HSA), ovalbumin, casein, gelatin, or a salt thereof), a surfactant, powdered skim milk, or the like, and then performing blocking treatment (masking treatment) for the solid phase carrier.

Examples of a labeling substance that can be used in enzyme immunoassay include peroxidase (POD), alkaline phosphatase (ALP), β-galactosidase, urease, catalase, glucose oxidase, lactate dehydrogenase, and amylase.

Also, examples of a labeling substance that can be used in fluorescence immunoassay include fluorescein isothiocyanate, tetramethylrhodamine isothiocyanate, substituted rhodamine isothiocyanate, and dichlorotriazine isothiocyanate.

Also, examples of a labeling substance that can be used in radioimmunoassay include tritium, iodine 125, and iodine 131.

Also, examples of a labeling substance that can be used in luminescence immunoassay include substances involved in reaction systems such as a NADH-FMNH₂-luciferase reaction system, a luminol-hydrogen peroxide-POD reaction system, an acridinium ester reaction system, and a dioxetane compound reaction system.

Regarding a method for binding of an antibody (or a antigen) such as the above "avian-derived anti-human HMGB1 antibody" with a labeling substance such as an enzyme, such binding can be performed by mixing and contacting an antibody (or an antigen) and a labeling substance with a bifunctional crosslinking reagent such as glutaraldehyde, carbodiimide, imide ester, maleimide, or the like, so as to react with amino groups, carboxyl groups, thiol groups, aldehyde groups, hydroxyl groups, or the like of each of the antibody (or the antigen) and the labeling substance according to a known method as described in the Japanese Society of Laboratory Medicine (Ed.), "Clinical Pathology, Extra Edition, No. 53, Immunoassay for Laboratory Tests -Techniques and Applications-," The Clinical Pathology Press, issued 1983; The Japanese Biochemical Society (Ed.), "New Biochemistry Experimental Lectures 1 (Shin Seikagaku Jikken Koza 1) Protein IV," TOKYO KAGAKU DOJIN CO., LTD, issued 1991, or the like, for example.

Procedures for measurement in the above immunoassay method such as enzyme immunoassay, fluorescence immunoassay, radioimmunoassay, or luminescence immunoassay can be performed according to a known method (Japanese Society of Laboratory Medicine (Ed.), "Clinical Pathology, Extra Edition, No. 53, Immunoassay for Laboratory Tests -Techniques and Applications-," The Clinical Pathology Press, issued 1983; Eiji Ishikawa et al., (Ed.) "Enzyme Immunoassay," Third Edition, IGAKU-SHOIN, issued 1987; Tsunehiro Kitagawa et al., (Ed.) "Protein, Nucleic Acid and Enzyme, Separate Volume No. 31, Enzyme Immunoassay," KYORITSU SHUPPAN CO., LTD, issued 1987), for example.

For example, a complex of "solid phase carrier-antibody" = "human HMGB1" = "antibody-labeling substance" is formed by reacting an immobilized antibody ("solid phase carrier-antibody") with a sample and at the same time, reacting a labeled antibody ("antibody-labeling substance") with the same, or reacting a labeled antibody with the same after washing.

Subsequently, unbound labeled antibodies are washed and separated and then the level of the labeled antibody indirectly binding to the solid phase carrier or the level of the unbound labeled antibody as a result of binding of "solid phase carrier-antibody" = "human HMGB1" = "antibody-labeling substance" is measured, so that the level (concentration) of human HMGB1 contained in the sample can be measured.

Specifically, when enzyme immunoassay is employed, an enzyme with which an antibody has been labeled is reacted with a substrate under the optimum conditions or the like and then the level of the enzyme reaction product is measured by an optical method or the like.

Also, when fluorescence immunoassay is employed, fluorescence intensity resulting from labeling with a fluorescent substance is measured.

Also, when radioimmunoassay is employed, radiation dose resulting from labeling with a radioactive substance is measured.

Also, when luminescence immunoassay is employed, the amount of luminescence resulting from a luminescent reaction system is measured.

### (5) Immunoassay method using agglutination reaction method

The immunoassay method of the present invention can be performed according to an immunoassay method for measuring the level (concentration) of human HMGB1 contained in a sample by measuring the generation of immune complex aggregates by an optical method based on the transmitted light or scattered light thereof, or visually measuring the same, such as immunonephelometry, latex turbidimetry, a latex agglutination method, a hemagglutination method, or a particle agglutination method.

When the above "avian-derived anti-human HMGB1 antibody" is immobilized on a solid phase carrier and then used, examples of a solid phase carrier that can be used herein include particles made of material such as polystyrene, a styrene-styrene sulfonate copolymer, an acrylonitrile-butadiene-styrene copolymer, a vinyl chloride-acrylic ester copolymer, a vinyl acetate-acrylic acid copolymer, polyacrolein, a styrene-methacrylic acid copolymer, a styrene-glycidyl(meth)acrylate copolymer, a styrene-butadiene copolymer, a methacrylic acid polymer, an acrylic acid polymer, latex, gelatin, a liposome, a microcapsule, an erythrocyte, silica, alumina, carbon black, a metal compound, ceramics, metal, or a magnetic material.

The above "avian-derived anti-human HMGB1 antibody" can be immobilized on a solid phase carrier by a known method such as physical adsorption, chemical binding, or a combination thereof.

When physical adsorption is employed, immobilization can be performed according to a known method that involves mixing and contacting an antibody with a solid phase carrier in a solution such as a buffer, or contacting an antibody dissolved in a buffer or the like with a solid phase carrier, for example.

Also, when chemical binding is employed, immobilization can be performed according to a known method as described in the Japanese Society of Laboratory Medicine (Ed.), "Clinical Pathology, Extra Edition, No. 53, Immunoassay for Laboratory Tests -Techniques and Applications-," The Clinical Pathology Press, issued 1983; The Japanese Biochemical Society (Ed.), "New Biochemistry Experimental Lectures 1 (Shin Seikagaku Jikken Koza 1) Protein IV," TOKYO KAGAKU DOJIN CO., LTD, issued 1991, or the like by mixing and contacting an antibody and a solid phase carrier with a bifunctional crosslinking reagent such as glutaraldehyde, carbodiimide, imide ester, or maleimide, so as to react with amino groups, carboxyl groups, thiol groups, aldehyde groups, hydroxyl groups, or the like of each of the antibody and the solid phase carrier, for example.

Also, if there is a need to perform treatment for suppressing non-specific reactions, natural aggregation of a solid phase carrier, or the like, the surface or inner wall surface of a solid phase carrier on which the above "avian-derived anti-human HMGB1 antibody" has been immobilized is treated by a known method that involves contacting and coating the same with a protein (e.g., bovine serum albumin (BSA), human serum albumin (HSA), ovalbumin, casein, gelatin, or a salt thereof), a surfactant, powdered skim milk, or the like, and then performing blocking treatment (masking treatment) for the solid phase carrier.

In addition, when latex turbidimetry is employed as a measurement principle, the particle size of latex particles to be used as solid phase carriers is not particularly limited. However, the average particle size of latex particles preferably ranges from 0.04 µm to 1 µm because of: the degree of aggregate formation as a result of the binding of latex particles via the above "avian-derived anti-human HMGB1 antibody" and a substance to be measured (human HMGB1); and the ease of measuring the thus formed aggregates, for example.

Also, when latex turbidimetry is employed as a measurement principle, no general description can be given concerning the content (concentration) of latex particles on which the above "avian-derived anti-human HMGB1 antibody" has been immobilized. This is because the optimal concentration thereof differs depending on various conditions such as the concentration of human HMGB1 contained in a sample, the distribution density of the above "avian-derived anti-human HMGB1 antibody" on latex particle surfaces, the particle size of latex particles, and the mixing ratio of a sample to a measuring reagent.

However, in general, the concentration of the latex particles on which the above "avian-derived anti-human HMGB1 antibody" has been immobilized is generally adjusted to 0.005% (w/v) to 1% (w/v) in the reaction mixture when a sample is mixed with a measuring reagent, following which an antigen-antibody reaction of the above "avian-derived anti-human HMGB1 antibody" immobilized on latex particles with "human HMGB1" contained in the sample is performed for measurement. In this case, "latex particles, on which the avian-derived anti-human HMGB1 antibody has been immobilized" are contained in a measuring reagent at a concentration that allows realization of the above-described concentration of latex particles in a reaction mixture.

Furthermore, when an indirect agglutination method such as a latex agglutination method, a hemagglutination method, or a particle agglutination method is employed as a measurement principle, the particle size of particles to be used as solid phase carriers is not particularly limited. The average particle size preferably ranges from 0.01 µm to 100 µm and more preferably ranges from 0.5 µm to 10 µm.

Moreover, the specific gravity of these particles preferably ranges from 1 to 10 and more preferably ranges from 1 to 2.

In addition, examples of a container to be used for measurement when an indirect agglutination method such as a principle latex agglutination method, a hemagglutination method, or a particle agglutination method is employed as a measurement principle include test tubes, microplates (microtiter plates) and trays made of glass, polystyrene, polyvinyl chloride, polymethacrylate, or the like.

The bottom of each of these containers is preferably U-shaped, V-shaped, UV-shaped, or the like, sloping from the bottom center toward the periphery.

When the immunoassay method of the present invention is performed according to an immunoassay method such as an immunonephelometry method, a latex turbidimetry method, a latex agglutination method, a hemagglutination method, or a particle agglutination method, a phosphate buffer, a glycine buffer, a tris buffer, or Good's buffer, or the like can be used as a solvent. Furthermore, a reaction accelerator such as polyethylene glycol or a non specific reaction inhibitor may also be contained.

Procedures for measurement in the above immunoassay method such as immunonephelometry, latex turbidimetry, a latex agglutination method, a hemagglutination method, or a particle agglutination method can be performed by a known method or the like. For example, when measurement is performed by an optical method, a sample is reacted with the above "avian-derived anti-human HMGB1 antibody" or a sample is reacted with the above "avian-derived anti-human HMGB1 antibody" immobilized on a solid phase carrier and then transmitted light or scattered light is measured by end point assay or rate assay.

Also, when visual measurement is performed, in the above container such as a plate or a microplate, a sample is reacted with the above "avian-derived anti-human HMGB1 antibody" immobilized on a solid phase carrier and then the state of aggregation is visually determined.

In addition, measurement may also be performed using an apparatus such as a microplate reader instead of such visual measurement.

In addition, when the immunoassay method of the present invention is performed according to the above method for measuring the generation of immune complex aggregates, the use of the above "avian-derived anti-human HMGB1 antibody" as an antibody specifically binding to HMGB1 is essential. However, in addition to this antibody, an antibody specifically binding to another HMGB1 may also be used herein.

### [III] Immunoassay reagent for human HMGB1 in sample

### (1) General statement

The immunoassay reagent for human HMGB1 in a sample of the present invention (hereinafter may also be referred to as "the immunoassay reagent of the present invention" or "the measuring reagent of the present invention") is an immunoassay reagent for measuring human HMGB1 in a sample using an antigen-antibody reaction with an antibody specifically binding to human HMGB1. The immunoassay reagent is characterized by containing "an avian-derived anti-human HMGB1 antibody comprising an avian-derived antibody specifically binding to the amino acid sequence shown by the following formula (I): Lys Pro Asp Ala Ala Lys Lys Gly Val Val Lys Ala Glu Lys Ser (I) of human HMGB1" ("avian-derived anti-human HMGB1 antibody").

Specifically, the immunoassay reagent for human HMGB1 in a sample is **characterized in that** the above "avian-derived anti-human HMGB1 antibody" is used as an antibody specifically binding to human HMGB1 that is a substance to be measured and the "avian-derived anti-human HMGB1 antibody" is contained in the immunoassay reagent.

The immunoassay reagent of the present invention contains the above "avian-derived anti-human HMGB1 antibody"; that is, a high-titer antibody having high capability of binding to human HMGB1, so that it is a highly sensitive immunoassay reagent capable of accurately measuring even trace amounts of human HMGB1 contained in a sample.

Also, the immunoassay reagent of the present invention contains the above "avian-derived anti-human HMGB1 antibody"; that is, a high-titer antibody (used therein) that has high capability of binding to human HMGB1 and can be obtained with high probability. The immunoassay reagent has a small possibility of producing rejected products (defective products) with low measuring sensitivity, but can be produced with good production yields, so that the immunoassay reagent has improved productivity thereof.

The "avian-derived anti-human HMGB1 antibody" can be used without particular restrictions, as long as it is such an antibody, by which the above effects can be obtained.

In addition, when the immunoassay reagent contains two or more types of antibody specifically binding to human HMGB1, at least one of the two or more antibodies is the "avian-derived anti-human HMGB1 antibody."

Furthermore, the other antibody (antibodies) may be any antibody as long as it is an "antibody specifically binding to human HMGB1."

In addition, of the two or more types of antibody, 2 or more antibodies may be "avian-derived anti-human HMGB1 antibodies" or all antibodies to be used herein may be the "avian-derived anti-human HMGB1 antibodies."

Any measurement principle can be applied without particular restrictions for the immunoassay reagent of the present invention. Examples of such measurement principle include a measurement principle of an immunoassay method (e.g., a sandwich method or a competitive method) using a labeling substance, such as enzyme immunoassay, fluorescence immunoassay, radioimmunoassay, or luminescence immunoassay, and a measurement principle of an immunoassay method for measuring the generation of immune complex aggregates, such as immunonephelometry, latex turbidimetry, a latex agglutination method, a hemagglutination method, or a particle agglutination method.

For example, in the case of an immunoassay reagent for which a sandwich method employed for enzyme immunoassay (ELISA), fluorescence immunoassay, luminescence immunoassay, or the like is employed as a measurement principle, either or both of a labeled antibody prepared by binding a labeling substance to the antibody and an immobilized antibody prepared by immobilizing the antibody on a solid phase carrier are the above "avian-derived anti-human HMGB1 antibodies." Moreover, both antibodies may be the above "avian-derived anti-human HMGB1 antibodies."

For example, in the case of an immunoassay reagent for which latex turbidimetry, a latex agglutination method, a hemagglutination method, a particle agglutination method, or the like is employed as a measurement principle, an antibody to be immobilized on a solid phase carrier such as a latex particle is "the above avian-derived anti-human HMGB1 antibody." Furthermore, in the case of a measuring reagent for which immunonephelometry is employed as a measurement principle, the above "avian-derived anti-human HMGB1 antibody" may be used as an antibody.

The above "avian-derived anti-human HMGB1 antibody" may be any antibody as long as it is an avian-derived antibody.

Examples of birds to be used herein include chickens, quails, pheasants, ostriches, and ducks. Birds of the family *Phasianidae* are preferred and chickens are particularly preferred.

In addition, the immunoassay reagent of the present invention is characterized by containing the above "avian-derived anti-human HMGB1 antibody." Hence, details concerning the "avian-derived anti-human HMGB1 antibody" contained in the immunoassay reagent of the present invention are as described in the above section, "[I] Avian-derived anti-human HMGB1 antibody" and the like. Moreover, details concerning measurement principles and the like for the immunoassay reagent of the present invention are as described in the above section, "[II] Immunoassay method for human HMGB1 in a sample," and the like.

### (2) Other ingredients of reagent

In the immunoassay reagent of the present invention, various aqueous solvents can be used.

Examples of such aqueous solvents include purified water, normal saline, and various buffers such as a tris buffer, a phosphate buffer, and phosphate buffered saline.

Regarding pH of such buffer, an appropriate pH can be adequately selected and used and is not particularly limited. In general, the pH is selected from the range between pH 3 and 12 and then used.

The immunoassay reagent of the present invention may adequately contain: one, two, or more types of a protein such as bovine serum albumin (BSA), human serum albumin (HSA), ovalbumin, casein, gelatin, or a salt thereof; various salts; various saccharides; powdered skim milk; various animal sera such as normal rabbit serum; various antiseptics such as sodium azide or an antibiotic; an activator; a reaction accelerating substance; a sensitivity-increasing substance such as polyethylene glycol; a non specific reaction-inhibiting substance; or various surfactants such as a nonionic surfactant, an amphoteric surfactant, and an anionic surfactant, in addition to ingredients of a reagent, such as an "immobilized antibody" that is an antibody (e.g., the above "avian-derived anti-human HMGB1 antibody") immobilized on a solid phase carrier and/or a "labeled antibody" prepared by binding a labeling substance such as an enzyme to an antibody (e.g., the above "avian-derived anti-human HMGB1 antibody").

Furthermore, the concentration (content) of each of these ingredients in the measuring reagent is not particularly limited and preferably ranges from 0.001% (W/V) to 10% (W/V) and particularly preferably ranges from 0.01 % (W/V) to 5% (W/V).

In addition, examples of the above surfactants include: nonionic surfactants such as a sorbitan fatty acid ester, a glycerine fatty acid ester, a decaglycerine fatty acid ester, a polyoxyethylene sorbitan fatty acid ester, a polyoxyethylene glycerine fatty acid ester, a polyethylene glycol fatty acid ester, a polyoxyethylene alkyl ether, polyoxyethylene phytosterol, phytostanol, a polyoxyethylene polyoxypropylene alkyl ether, a polyoxyethylene alkylphenyl ether, polyoxyethylene castor oil, hydrogenated castor oil, or polyoxyethylene lanolin; amphoteric surfactants such as acetic acid betaine; and anionic surfactants such as polyoxyethylene alkyl ether sulfate or polyoxyethylene alkyl ether acetate.

### (3) Composition of measuring reagent

The immunoassay reagent of the present invention can be used independently for measurement of human HMGB1 in a sample. The immunoassay reagent can also be marketed alone.

Furthermore, the immunoassay reagent of the present invention can also be used in combination with other reagents for measurement of human HMGB1 in a sample. The immunoassay reagent can also be marketed in combination with other reagents.

Examples of such other reagents include a buffer, a diluent for a sample, a diluent for a reagent, a reagent containing a labeling substance, a reagent containing a substance that produces signals of color development or the like, a reagent containing a substance involved in production of signals of color development or the like, a reagent containing a substance for calibration, and a reagent containing a substance for accuracy control.

Moreover, the above "other" reagent(s) can be used as a 1st reagent and the immunoassay reagent of the present invention can be used as a 2nd reagent or the immunoassay reagent of the present invention can be used as a 1st reagent and the above "other" reagent(s) can be used as a 2nd reagent. In this manner, these reagents can be adequately used and marketed in various combinations.

### [IV] Method for obtaining avian-derived anti-human HMGB1 antibody

The method for obtaining the avian-derived anti-human HMGB1 antibody of the present invention is characterized by, upon obtainment of the avian-derived anti-human HMGB1 antibody, immunizing birds with a peptide comprising the amino acid sequence shown by the following formula (I): Lys Pro Asp Ala Ala Lys Lys Gly Val Val Lys Ala Glu Lys Ser (I) of human HMGB1, a peptide comprising an amino acid sequence obtained by subjecting the amino acid sequence shown by formula (I) to deletion, substitution, insertion, addition, or modification of one to several amino acid residues, or a conjugate of the peptide and a carrier as an immunogen.

In addition, the number of the above "several" amino acid residues to be deleted, substituted, inserted, added, or modified generally ranges from 1 to 4, preferably ranges from 1 to 3, further preferably ranges from 1 to 2, and is particularly preferably 1.

According to the method for obtaining the avian-derived anti-human HMGB1 antibody of the present invention, which is characterized by immunizing birds with a peptide comprising the amino acid sequence shown by the above formula (I), a peptide comprising an amino acid sequence obtained by subjecting the amino acid sequence shown by formula (I) to deletion, substitution, insertion, addition, or modification of one to several amino acid residues, or a conjugate of the peptide and a carrier as an immunogen, the thus obtained "avian-derived anti-human HMGB1 antibody" can specifically bind to the above amino acid sequence shown by formula (I).

In the method for obtaining the avian-derived anti-human HMGB1 antibody of the present invention, the above amino acid sequence shown by formula (I) corresponds to a part of the amino acid sequence of human HMGB1 having low homology with the amino acid sequence of avian HMGB1. Hence, the "avian-derived anti-human HMGB1 antibody" obtained by immunizing birds with the above immunogen has the following two characteristics.
(a) The avian-derived anti-human HMGB1 antibody can specifically bind to human HMGB1.
(b) The avian-derived anti-human HMGB1 antibody cannot bind (or binds with difficulty) to HMGB1 generated *in vivo* in the birds as a result of immunization with the above immunogen. Thus, no avian-derived anti-human HMGB1 antibody binds to HMGB1 generated *in vivo* in the above birds (or the number of the avian-derived anti-human HMGB1 antibody binding to such HMGB1 is low). Accordingly, the avian-derived anti-human HMGB1 antibody is a high-titer antibody having high capability of binding to human HMGB1 and can be obtained with high probability.

Therefore, according to the method for obtaining the avian-derived anti-human HMGB1 antibody of the present invention, the thus obtained "avian-derived anti-human HMGB1 antibody" is a high-titer antibody having high capability of binding to human HMGB1 and the avian-derived anti-human HMGB1 antibody can be obtained with high probability. Hence, the productivity of the avian-derived anti-human HMGB1 antibody is high.

In addition, as an immunogen for obtaining the "avian-derived anti-human HMGB1 antibody" of the present invention, a peptide comprising the amino acid sequence shown by the following formula (I): Lys Pro Asp Ala Ala Lys Lys Gly Val Val Lys Ala Glu Lys Ser (I) or a conjugate of the peptide comprising the amino acid sequence shown by formula (I) and a carrier is preferred. Particularly a conjugate of the peptide comprising the above amino acid sequence shown by formula (I) and a carrier is preferred.

In the method for obtaining the avian-derived anti-human HMGB1 antibody of the present invention, animals to be immunized with the above immunogen are birds.

Examples of the birds include chickens, quails, pheasants, ostriches, and ducks. Birds of the family *Phasianidae* are preferred and chickens are particularly preferred.

In addition, regarding the method for obtaining the avian-derived anti-human HMGB1 antibody of the present invention, details concerning the avian-derived anti-human HMGB1 antibody, its immunogens, and the method for obtaining the avian-derived anti-human HMGB1 antibody are as described in the above section, "[I] Avian-derived anti-human HMGB1 antibody" and the like.

### [V] Method for improving productivity for obtaining anti-human HMGB1 antibody

The method for improving productivity for obtaining the anti-human HMGB1 antibody of the present invention is characterized by, upon obtainment of the anti-human HMGB1 antibody, immunizing birds with a peptide comprising the amino acid sequence shown by the following formula (I): Lys Pro Asp Ala Ala Lys Lys Gly Val Val Lys Ala Glu Lys Ser (I) of human HMGB1, a peptide comprising an amino acid sequence obtained by subjecting the amino acid sequence shown by formula (I) to deletion, substitution, insertion, addition, or modification of one to several amino acid residues, or a conjugate of the peptide and a carrier as an immunogen.

In addition, the number of the above "several" amino acid residues to be deleted, substituted, inserted, added, or modified generally ranges from 1 to 4, preferably ranges from 1 to 3, further preferably ranges from 1 to 2, and is particularly preferably 1.

According to the method for improving productivity for obtaining the anti-human HMGB1 antibody of the present invention, which is characterized by immunizing birds with a peptide comprising the above amino acid sequence shown by formula (I), a peptide comprising an amino acid sequence obtained by subjecting the amino acid sequence shown by formula (I) to deletion, substitution, insertion, addition, or modification of one to several amino acid residues, or a conjugate of the peptide and a carrier as an immunogen, the thus obtained avian-derived "anti-human HMGB1 antibody" can specifically bind to the above amino acid sequence shown by formula (I).

In the method for improving productivity for obtaining the anti-human HMGB1 antibody of the present invention, the above amino acid sequence shown by formula (I) corresponds to a part of the amino acid sequence of human HMGB1 having low homology with the amino acid sequence of avian HMGB1. Hence, the "anti-human HMGB1 antibody" obtained by immunizing birds with the above immunogen has the following two characteristics.
(a) The anti-human HMGB1 antibody can specifically bind to human HMGB1.
(b) The anti-human HMGB1 antibody cannot bind (or binds with difficulty) to HMGB1 generated *in vivo* in the birds as a result of immunization with the above immunogen. Thus, no avian-derived anti-human HMGB1 antibody binds to HMGB1 generated *in vivo* in the above birds (or the number of the avian-derived anti-human HMGB1 antibody binding to such HMGB1 is low). Accordingly, the anti-human HMGB1 antibody is a high-titer antibody having high capability of binding to human HMGB1 and can be obtained with high probability.

Therefore, according to the method for improving productivity for obtaining the anti-human HMGB1 antibody of the present invention, the thus obtained avian-derived "anti-human HMGB1 antibody" is a high-titer antibody having high capability of binding to human HMGB1 and the anti-human HMGB1 antibody can be obtained with high probability.

Specifically, according to the present invention, a possibility of production of a low-titer anti-human HMGB1 antibody having low capability of binding to human HMGB1 is extremely low, so that the production yield of the high-titer anti-human HMGB1 antibody can be significantly improved.

In addition, as an immunogen for obtaining the "anti-human HMGB1 antibody" of the present invention, a peptide comprising the amino acid sequence shown by the following formula (I): Lys Pro Asp Ala Ala Lys Lys Gly Val Val Lys Ala Glu Lys Ser (I) or a conjugate of the peptide comprising the amino acid sequence shown by formula (I) and a carrier is preferred. Particularly, a conjugate of the peptide comprising the above amino acid sequence shown by formula (I) and a carrier is preferred.

In the method for improving productivity for obtaining the anti-human HMGB1 antibody of the present invention, animals to be immunized with the above immunogen are birds.

Examples of the birds include chickens, quails, pheasants, ostriches, and ducks. Birds of the family *Phasianidae* are preferred and chickens are particularly preferred.

In addition, regarding the method for improving productivity for obtaining the anti-human HMGB1 antibody of the present invention, details concerning the avian-derived anti-human HMGB1 antibody, its immunogens, the method for obtaining the avian-derived anti-human HMGB1 antibody, and the like are as described in the above section, "[I] Avian-derived anti-human HMGB1 antibody" and the like.

### Examples

The present invention will be described in detail by examples as follows, but the present invention is not limited by these examples.

### [Example 1] (Selection of an amino acid sequence of human HMGB1 having low homology with the amino acid sequence of chicken HMGB1)

An amino acid sequence having low homology with the amino acid sequence of avian (chicken) HMGB1 was selected from the amino acid sequence of human HMGB1.
(1) The amino acid sequence of human HMGB1 according to Wen et al., [Wen et al., Nucleic Acids Res. (1989), vol. 17, pp. 1197-1214] was compared with the amino acid sequence of avian (chicken) HMGB1 according to Caldwell et al., Luw et al., and Lee et al., [Caldwell et al., Genome Biol. (2005), Vol. 6, No. 1, R5; Luw et al., Biochim. Biophys. Acta (2000), vol. 1493, Nos. 1-2, pp. 64-72; Lee et al., Gene (1998), vol. 225, Nos. 1-2, pp. 97-105].
(2) From this comparison, "Lys Pro Asp Ala Ala Lys Lys Gly Val Val Lys Ala Glu Lys Ser" [formula (I)] , which corresponds to the sequence of human HMGB1 from 166th (Lys) to 180th (Ser) from the N-terminus, was selected as an amino acid sequence of human HMGB1 with low homology to that of the amino acid sequence of chicken HMGB1.

In addition, the amino acid sequence of the formula (I), "Lys Pro Asp Ala Ala Lys Lys Gly Val Val Lys Ala Glu Lys Ser" [formula (I)] differed from the corresponding amino acid sequence, "Lys Ser Gly Ala Glu Lys Lys Gly Pro Gly Arg Pro Thr Gly" of human HMGB2 by 9 out of a total of 15 amino acid residues.

### [Example 2] (Synthesis of a peptide of the selected amino acid sequence)

A peptide, "Cys Lys Pro Asp Ala Ala Lys Lys Gly Val Val Lys Ala Glu Lys Ser" [formula (II)], was synthesized, with a carrier binding Cystein to the N-terminal end of the selected amino acid sequence in Example 1, "Lys Pro Asp Ala Ala Lys Lys Gly Val Val Lys Ala Glu Lys Ser" (formula (I)).

First, the peptide comprising the amino acid sequence "Cys Lys Pro Asp Ala Ala Lys Lys Gly Val Val Lys Ala Glu Lys Ser" [formula (II)] was synthesized according to the instruction manual of Model 430A Automatic Peptide Synthesizer (Applied Biosystems) using the t-butoxycarbonyl amino acid solid phase method.

To suppress side reactions, the synthesized peptide was cleaved from resin by the hydrogen fluoride method in the presence of dimethyl sulphide, p-thiocresol, m-cresol, and anisole as scavengers.

Subsequently, scavengers were extracted with dimethyl ether and then the synthesized peptides were extracted with 2N acetic acid.

The peptide was purified first by anion exchange chromatography using anion exchange resin (DOWEX 1-X2). Main peak patterns were confirmed by high performance liquid chromatography (HPLC) using an octadecyl (ODS) column.

After freeze-drying and concentration using an evaporator, the peptide was further purified by HPLC and isolated.

JASCO TWINCLE pump with GP-A40 gradient mixer (JASCO) was used for HPLC. Elution at 210 nm, 1.28 AUFS was monitored with UVIDEC-100V (JASCO). The peptide was purified using a 0 - 70% acetonitrile gradient with 0.1 % trifluoroacetic acid (TFA) at a flow rate of 7.0 mL/min on a YMC-D-ODS-5 reverse phase ODS column (20 mm × 300 mm, YMC Co., Ltd.)

The purified and isolated synthetic peptide was freeze-dried and concentrated using an evaporator.

Next, the purity of the obtained synthetic peptide was analyzed by HPLC.

JASCO TWINCLE pump with GP-A40 gradient mixer (JASCO) was used for HPLC. Elution at 210 nm, 1.28 AUFS was monitored with UVIDEC-100V (JASCO). The peptide was purified using a 25 min, 0 - 70% acetonitrile gradient with 0.1% trifluoroacetic acid (TFA) at a flow rate of 1.0 mL/min on a YMC-R-ODS-5 reverse phase ODS column (4.9 mm × 300 mm, YMC Co., Ltd.)

The analysis showed that the purity of the obtained synthetic peptide was almost 100%. [Example 3] (Preparation of conjugates of the selected peptide sequence and a carrier)

Two (2) types of conjugates, comprising of the selected peptide sequence synthesized in Example 2 "Cys Lys Pro Asp Ala Ala Lys Lys Gly Val Val Lys Ala Glu Lys Ser" [formula (II)] and one of two carriers, were prepared.

### (1) Preparation of a conjugate of a carrier, bovine serum albumin and the synthetic peptide

Ten (10) mg of bovine serum albumin (BSA) [Seikagaku Corporation] as a carrier was dissolved in 10 mM potassium dihydrogenphosphate-dipotassium hydrogen phosphate buffer (pH 7.0). 150 µL of 2.5% maleimidobenzoyl-N-hydroxysuccinimide ester (MBS) (Pierce) solution in N,N-dimethylformamide was added to the BSA solution and stirred for 30 minutes at room temperature.

The reaction mixture was loaded on a Sephadex G-25 gel filtration column (Pharmacia-LKB) equilibrated in advance with 10 mM potassium dihydrogen phosphate-dipotassium hydrogen phosphate buffer (pH 7.0) at 4°C. Absorbance was monitored at 280 nm and then the MBS-carrier complex was isolated

The pH of MBS-carrier complex was adjusted to 7.0 with trisodium phosphate. The peptide comprising the amino acid sequence of formula (II) "Cys Lys Pro Asp Ala Ala Lys Lys Gly Val Val Lys Ala Glu Lys Ser" synthesized in Example 2 was added to the MBS-carrier complex and incubated for 150 minutes.

After the reaction, the resultant was dialyzed against water 3 times and then freeze dried. A conjugate of a carrier bound to the above peptide; that is, a conjugate of the peptide comprising the amino acid sequence of formula (II) "Cys Lys Pro Asp Ala Ala Lys Lys Gly Val Val Lys Ala Glu Lys Ser" and BSA (the carrier) was obtained.

### (2) Preparation of a conjugate of a carrier, Keyhole Limpet hemocyanin and the synthetic peptide

Ten (10) mg of Keyhole Limpet hemocyanin (KLH) (Calbiochem) as a carrier was dissolved in 10 mM potassium dihydrogenphosphate-dipotassium hydrogen phosphate buffer (pH 7.0). 150 µL of 2.5% maleimidobenzoyl-N-hydroxysuccinimide ester (MBS) (Pierce) solution in N,N-dimethylformamide was added to the KLH solution and stirred for 30 minutes at room temperature.

The reaction mixture was loaded on a Sephadex G-25 gel filtration column (Pharmacia-LKB) equilibrated in advance with 10 mM potassium dihydrogen phosphate-dipotassium hydrogen phosphate buffer (pH 7.0) at 4°C. Absorbance was monitored at 280 nm and then the MBS-carrier complex was isolated.

The pH of MBS-carrier complex was adjusted to 7.0 with trisodium phosphate. The peptide comprising the amino acid sequence of formula (II) "Cys Lys Pro Asp Ala Ala Lys Lys Gly Val Val Lys Ala Glu Lys Ser" synthesized in Example 2 was added to the MBS-carrier complex and incubated for 150 minutes.

After the reaction, the resultant was dialyzed against water 3 times and then freeze dried. A conjugate of a carrier bound to the above peptide; that is, a conjugate of the peptide comprising the amino acid sequence of formula (II) "Cys Lys Pro Asp Ala Ala Lys Lys Gly Val Val Lys Ala Glu Lys Ser" and KLH (the carrier) was obtained.

### [Example 4] (Preparation of human HMGB1 and human HMGB2)

Human HMGB1 and human HMGB2 were prepared from HL60 cells according to the method by Cabart et al., [P. Cabart et al., Cell Biochemistry and Function 13 (1995), pp. 125-133].
(1) First, HL60 cells were cultured in 300 mL of RPMI 1640 medium (Gibco) containing inactivated 10% fetal calf serum (FCS) (Gibco) for 1 week.
(2) Next, the cultured HL60 cells were collected, washed with RPMI 1640 medium, and then cultured in 3 L of PFHM-II serum- and·protein-free medium (Invitrogen Corporation) for 2 weeks.
(3) Subsequently, the culture supernatant was run through a heparin-sepharose column (Sigma) equilibrated with phosphate buffered saline (PBS).
   After thorough washing with PBS, proteins were eluted with PBS containing 0.5 M sodium chloride.
   Elution was monitored by absorbance at 280 nm. Fractions having absorbance at 280 nm were collected.
(4) These fractions having absorbance at 280 nm (that is, fractions containing proteins) were thoroughly dialyzed using 5 mM borate buffer (pH 9.0) containing 0.2 M sodium chloride.
(5) Next, the resultant was run through a CM-Sephadex G25 column (Pharmacia-LKB) equilibrated with 7.5 mM borate buffer (pH 9.0).
   After thorough washing with 7.5 mM borate buffer (pH 9.0), the proteins were eluted with 7.5 mM sodium borate buffer (pH 9.0) containing 200 mM sodium chloride, followed by cation exchange chromatography.
(6) Each fraction above was subjected to SDS-polyacrylamide electrophoresis. Fractions containing human HMGB1 and human HMGB2 were identified based on the mobility of the electrophoresis.
   Human HMGB1 and human HMGB2 were each prepared from human cells (HL60 cells) by the above procedures.

### [Example 5] (Preparation of an avian-derived anti-human HMGB1 antibody and a mammal-derived anti-human HMGB1 antibody using conjugates of synthesized peptides and carriers as immunogens)

An avian-derived anti-human HMGB1 antibody was prepared using "a conjugate of a synthesized peptide and a carrier" prepared in Example 3 as an immunogen.

Also, as a control, a mammal-derived anti-human HMGB1 antibody was prepared using "a conjugate of a synthesized peptide and a carrier" prepared in Example 3 as an immunogen.

### [1] Preparation of avian-derived anti-human HMGB1 antibodies

Chickens (birds) were immunized with "a conjugate of a synthesized peptide and a carrier" prepared in Example 3 as an immunogen. An anti-human HMGB1 antibody was obtained from the chickens.
(1) The conjugate of the peptide, comprised of the amino acid sequence of formula (II), "Cys Lys Pro Asp Ala Ala Lys Lys Gly Val Val Lys Ala Glu Lys Ser" prepared in Example 3, and BSA (the carrier) was dissolved in saline (0.9% sodium chloride aqueous solution) to a concentration of 100 µg/mL.
   Next, the solution and Freund's complete adjuvant were mixed in equivalent amounts to result in an emulsion. Ten (10) hens (AGC TECHNO GLASS CO., LTD.) were immunized with the emulsion as an immunogen. Specifically, 0.5 mL of the immunogen was injected into the roots of a wing of each hen.
(2) Two (2) weeks after the first immunization, the above immunogen was dissolved in saline to a concentration of 100 µg/mL. The solution and Freund's incomplete adjuvant were mixed in equivalent amounts to result in an emulsion. A booster injection of 0.5 mL of the emulsion was given to the animal.
   Booster injections were repeated at intervals of 2 weeks.
(3) Then, antibody titers in the yolks of eggs laid by the 10 hens, which were the immunized animals, were measured by enzyme linked immunosorbent assay (ELISA) every week after week 6 of the first immunization.

ELISA were performed as described in (i) to (vi) below.
(i) The conjugate of the peptide comprised of the amino acid sequence of formula (II) "Cys Lys Pro Asp Ala Ala Lys Lys Gly Val Val Lys Ala Glu Lys Ser" and KLH (the carrier) prepared in Example 3 was dissolved in saline to a concentration of 1 µg/mL. The conjugate was immobilized by adding 100 µL each of the solution to a 96-well microplate (Nunc) and then let stand at 37°C for 2 hours.
(ii) Next, each well of the microplate was washed with wash solution with the composition described below.
   Wash solution: 0.05% Tween 20 in phosphate buffered saline.
   Phosphate buffered saline (PBS): an aqueous solution of 5.59 mM disodium hydrogen phosphate, 1.47 mM potassium dihydrogen phosphate, 137 mM sodium chloride, and 2.68 mM potassium chloride, pH adjusted to 7.2.
   For blocking, 10 mM potassium dihydrogen phosphate-dipotassium hydrogen phosphate buffer (pH 7.2) with 1% BSA was added in aliquots of 300 µL per well and let stand at 37°C for 2 hours, followed by wash with the wash solution.
(iii) Next, the egg yolks were tested for production of anti-human HMGB1 antibody. 100 µL of the egg yolks (laid by the aforementioned 10 hens) was dissolved in 900 µL of saline. The solution was diluted by 10-fold and then further diluted by 1,000, 10, 000 and 100,000-fold with saline.
   The diluted solutions of the egg yolks were added in amounts of 100 µL per well to the microplate of (ii) above, let stand at 37°C for 2 hours for reaction, and then washed with the wash solution.
(iv) As a control, in the place of the above diluted yolk solutions, 100 µL each of PBS containing 1% BSA was added. The resultant was let stand at 37°C for 2 hours and then washed with the wash solution.
(v) An anti-chicken IgY antibody (Up-Date) labeled with peroxidase (POD) was diluted by 5,000 fold with PBS containing 3% BSA. The solution was added in amounts of 100 µL per well to the microplate in (iii) and (iv) above and then the microplate was let stand at 37°C for 2 hours for reaction.
(vi) Next, each well of the microplates was washed with the wash solution and then POD reaction solution was added in 100 µL aliquots per well, followed by incubation at room temperature. POD reaction solution was prepared by adding 2 µL of 1.7% hydrogen peroxide to 1 mL of a 50 mM disodium hydrogen phosphate-24 mM citrate buffer containing 3 mM 2,2'-azino-bis(3-ethyl benzothiazoline-6-sulfonic acid) [ABTS] immediately before use.

Subsequently 150 µL of 6 N sulfuric acid was added to each well after 15 minutes to stop the reaction.

The absorbance of each well was measured at 415 nm using an EIA plate reader (Bio-Rad Laboratories Inc.).
(4) From the result of antibody titer assay of the egg yolks in (3) above, it was confirmed that the antibody titers reached a plateau 1.5 to 2 months after the first immunization. An antibody (IgY) was obtained from the yolk of every egg laid by the 10 immunized hens.
(5) Forty (40) mL of 10 mM Tris hydrochloride buffer (pH 7.4) containing 140 mM sodium chloride and 0.01% sodium azide was added to 10 mL of the yolk. The mixture was stirred well and then centrifuged to obtain supernatant.
(6) Next, to the supernatant, 7.5 mL of Tris hydrochloride buffer of (5) above containing 1 M calcium chloride and 3 mL of Tris hydrochloride buffer of (5) above containing 10% (W/V) dextran sulfate were added. After 30 minutes of stirring, the mixture was centrifuged to obtain supernatant and precipitate.

The supernatant was set aside. The precipitate was subjected to re-extraction with the Tris hydrochloride buffer of (5) above and the suspension was centrifuged.

The supernatant obtained by re-extraction was combined with the supernatant obtained before precipitation and re-extraction. The combined supernatant was diluted to 100 mL with the Tris hydrochloride buffer of (5) above.
(7) Twenty (20) g of anhydrous sodium sulfate was added to the solution. After 30 minutes of stirring, the suspension was centrifuged, and precipitate was isolated from supernatant by decantation. The precipitate was dissolved in 10 mL of PBS.
   Next, the resultant was dialyzed against PBS and then concentrated to obtain a globulin fraction.
(8) Next, the globulin fraction was run through a column in which the peptide synthesized in Example 2 had been immobilized, and the antibody was purified by affinity chromatography.

Affinity chromatography was performed as described below in (i) to (iv).
(i) Two (2) g of CNBr-sepharose (Pharmacia Biotech) was coupled with 10 mg of the peptide comprised of the amino acid sequence of formula (II) "Cys Lys Pro Asp Ala Ala Lys Lys Gly Val Val Lys Ala Glu Lys Ser" synthesized in Example 2, according to the instruction manual. An affinity chromatography column was prepared in which the above synthetic peptide had been immobilized.
(ii) The column was equilibrated in advance with PBS. Subsequently, the globulin fraction obtained in (7) above was run through the column.
(iii) The column was thoroughly washed with PBS and then a 0.1 M acetate buffer (pH 3.0) was run through the column.
(iv) Elution fractions were collected, dialyzed against PBS, and concentrated.

Through the above procedures of affinity chromatography, antibodies (polyclonal antibodies) that bind to the peptide comprised of the amino acid sequence of formula (II) "Cys Lys Pro Asp Ala Ala Lys Lys Gly Val Val Lys Ala Glu Lys Ser" were obtained from the 10 immunized hens.
(9) Each antibody (polyclonal antibody) obtained in (8) above from the 10 hens was subjected to confirmation of its binding capability to human HMGB1; that is, antibody titer.

Antibody titers were measured by ELISA as described in (i) to (vii) below.
(i) Human HMGB1 obtained in Example 4 was dissolved in saline to a concentration of 1 µg/mL. Human HMGB 1 was immobilized to the microplate by adding the solution in aliquots of 100 µL per well to a 96-well microplate (Nunc) and then let stand at 37°C for 2 hours.
(ii) Next, each well of the microplate was washed with the wash solution (PBS containing 0.05% Tween20, PBS's composition is described below).
   Phosphate buffered saline (PBS): an aqueous solution of 5.59 mM disodium hydrogen phosphate, 1.47 mM potassium dihydrogen phosphate, 137 mM sodium chloride, and 2.68 mM potassium chloride, pH adjusted to 7.2.
   Subsequently, 10 mM potassium dihydrogen phosphate-dipotassium hydrogen phosphate buffer (pH 7.2) containing 1% BSA was added in aliquots of 300 µL per well and then let stand at 37°C for 2 hours for blocking. Thereafter, the wells were washed again with the wash solution.
(iii) Next, the antibodies obtained in (8) above from the 10 hens were each diluted to concentrations of 0.01 µg/mL, 0.1 µg/mL, and 1 µg/mL with PBS.

The diluted antibodies were each added in aliquots of 100 µL per well to the microplate of (ii) above, let stand at 37°C for 2 hours for binding, and then washed with the wash solution.
(iv) In addition, as a control, 100 µL each of PBS containing 1% BSA was added in the place of the above diluted solutions of the antibodies. The wells were let stand at 37°C for 2 hours and then washed with the wash solution.
(v) An anti-chicken IgY antibody (Up-Date) labeled with POD was diluted by 5,000 fold with PBS containing 3% BSA. The solution was added in amounts of 100 µL per well to the microplate of (iii) and (iv) above and then let stand at 37°C for 2 hours for binding.
(vi) Next, each well of the microplates was washed with the wash solution. POD reaction solution was added in amounts of 100 µL per well, followed by reaction at room temperature. POD reaction solution was prepared by adding 2 µL of 1.7% hydrogen peroxide to 1 mL of 50 mM disodium hydrogen phosphate-24 mM citrate buffer containing 3 mM 3,3',5,5'-tetramethylbenzidine [TMBZ] immediately before use.

Fifteen (15) minutes later, 6N sulfuric acid was added in amounts of 150 µL per well to stop the reaction.

The absorbance of each well was measured at 450 nm using an EIA plate reader (Bio-Rad Laboratories Inc.).
(vii) Table 1 shows the results of measuring the absorbance of each antibody obtained from a total of 10 chickens.

| Antibody concentration (µg/mL) | Chicken 1 | Chicken 2 | Chicken 3 | Chicken 4 | Chicken 5 | Chicken 6 | Chicken 7 | Chicken 8 | Chicken 9 | Chicken 10 |
|---|---|---|---|---|---|---|---|---|---|---|
| 0.000 | 0.000 | 0.000 | 0.000 | 0.000 | 0.000 | 0.000 | 0.000 | 0.000 | 0.000 | 0.000 |
| 0.010 | 0.050 | 0.080 | 0.100 | 0.005 | 0.090 | 0.150 | 0.190 | 0.100 | 0.111 | 0.075 |
| 0.100 | 0.540 | 0.720 | 0.750 | 0.090 | 0.690 | 1.001 | 1.271 | 0.875 | 0.989 | 0.652 |
| 1.000 | 2.803 | 2.885 | 2.905 | 0.270 | 2.752 | 3.000 | 3.000 | 2.854 | 2.899 | 2.753 |

| | | | | | | | | | | |
|---|---|---|---|---|---|---|---|---|---|---|
| [Measured value is absorbance at 450 nm] | | | | | | | | | | |

As shown in Table 1, when the concentration of an antibody was 1 µg/mL, for example, for antibodies from 9 out of the 10 chickens were very high, reaching the absorbance of 2.7 or higher, suggesting the antibodies had very high binding capability to human HMGB1.

Specifically, in the case of the avian-derived anti-HMGB1 antibodies obtained by immunizing chickens (the birds) with the immunogen, it was confirmed that high-titer antibodies (antibodies having high binding capability to human HMGB1) can be obtained with a high probability of 9 out 10 immunized chickens.

### [2] Preparation of mammal-derived anti-human HMGB1 antibodies

As controls, rabbits, a mammal, were immunized with "a conjugate of a synthesized peptide and a carrier" prepared in Example 3 as an immunogen. An anti-human HMGB1 antibody was obtained from the rabbits.
(1) The conjugate of the peptide comprised of the amino acid sequence of formula (II) "Cys Lys Pro Asp Ala Ala Lys Lys Gly Val Val Lys Ala Glu Lys Ser" and BSA (the carrier) prepared in Example 3, was dissolved in saline (0.9% sodium chloride aqueous solution) to a concentration of 100 µg/mL.
Next, the solution and Freund's complete adjuvant were mixed in equivalent amounts to result in an emulsion. Ten (10) rabbits were then immunized with the emulsion as an immunogen. Specifically, 0.5 mL each of the immunogen was injected subcutaneously to the abdomen of each rabbit.
(2) Two (2) weeks after primary immunization, the above immunogen was dissolved in saline to a concentration of 100 µg/mL. The solution and Freund's incomplete adjuvant were mixed in equivalent amounts to result in an emulsion. A booster injection of 0.5 mL of the emulsion was given.
A booster injection was repeated every 2 weeks.
(3) Serum antibody titers of these 10 rabbits, the immunized animals, were measured by enzyme immunoassay (ELISA) every week after week 6 of primary immunization.
Procedures for ELISA were performed as described in the following (i) to (vi).
(i) The conjugate of the peptide comprised of the amino acid sequence of formula (II) "Cys Lys Pro Asp Ala Ala Lys Lys Gly Val Val Lys Ala Glu Lys Ser" and KLH (the carrier) prepared in Example 3, was dissolved in saline to a concentration of 1 µg/mL. The solution was added in amounts of 100 µL per well to a 96-well microplate (Nunc) and then let stand at 37°C for 2 hours. Thus, the conjugate of the peptide comprised of the amino acid sequence of formula (II) and KLH (the carrier) was immobilized in wells of the microplate.
(ii) Next, each well of the microplate was washed with the wash solution (phosphate buffered saline containing 0.05% Tween20 (an aqueous solution (pH 7.2) containing 5.59 mM disodium hydrogen phosphate, 1.47 mM potassium dihydrogen phosphate, 137 mM sodium chloride, and 2.68 mM potassium chloride)). Subsequently, a 10 mM potassium dihydrogen phosphate-dipotassium hydrogen phosphate buffer (pH 7.2) containing 1% BSA was added in amounts of 300 µL per well. The wells were let stand at 37°C for 2 hours for blocking and then washed again with the wash solution.
(iii) Next, 100 µL of the serum of the above 10 rabbits to be tested for antibody production against HMGB1 was dissolved in 900 µL of saline. The solution was diluted 10-fold and then it was separately diluted 1,000 fold, 10, 000 fold, and 100,000 fold with saline.
   Each diluted solution of the serum was added in amounts of 100 µL per well to the microplate of (ii) above and then let stand at 37°C for 2 hours for reaction. The wells were then washed with the wash solution.
(iv) Furthermore, as a control, 100 µL each of PBS containing 1% BSA was added instead of the diluted solution of the above serum and then let stand at 37°C for 2 hours. The wells were then washed with the wash solution.
(v) An anti-rabbit IgG antibody (Dako) labeled with peroxidase (POD) was diluted 5,000 fold with PBS containing 3% BSA. The solution was added in amounts of 100 µL per well to the microplate of (iii) above and that of (iv) above and then let stand at 37°C for 2 hours for reaction.
(vi) Next, each well of the microplates was washed with the wash solution. A POD reaction solution (prepared by adding 2 µL of 1.7% hydrogen peroxide to 1 mL of a 50 mM disodium hydrogen phosphate-24 mM citrate buffer containing 3 mM 2,2'-azino-bis(3-ethyl benzothiazoline-6-sulfonic acid) [ABTS], immediately before use) was added in amounts of 100 µL per well, followed by reaction at room temperature.

Subsequently, 15 minutes later, 6N sulfuric acid was added in amounts of 150 µL per well to stop reaction.
The absorbance of each well was measured at 415 nm using an EIA plate reader (Bio-Rad Laboratories Inc.).
(4) As a result of measuring antibody titers in the serum in (3) above, it was confirmed that the antibody titers reached a plateau 1.5 to 2 months after primary immunization. Thus, a serum (antiserum) was obtained from each of the 10 immunized rabbits.
(5) Anhydrous sodium sulfate (0.18 g) was added to 1 mL of the rabbit serum (antiserum) while stirring and keeping the solution at 22°C to 25°C.
After the anhydrous sodium sulfate had been dissolved, stirring at 22°C to 25°C was continued for 30 minutes.
(6) Next, the solution was centrifuged at 22°C to 25°C and 7,000 g for 10 minutes.
The precipitate obtained by centrifugation was dissolved in 1 mL of a 17.5 mM phosphate buffer (pH 6.3).
Next, the solution was sufficiently dialyzed with a 17.5 mM phosphate buffer (pH 6.3) and then concentrated.
(7) Thereafter, the concentrated product was run through a DEAE-cellulose column equilibrated in advance with a 17.5 mM phosphate buffer (pH 6.3).
Absorbance was measured in advance at 280 nm. Among flow through fractions, fractions having absorbance at 280 nm were fractionated as globulin fractions.
(8) Next, the globulin fractions were run through a column in which the peptide synthesized in Example 2 had been immobilized so that affinity chromatography was performed.
Affinity chromatography was performed as described in the following (i) to (iv).
(i) Two (2) g of CNBr-sepharose (Pharmacia Biotech) was reacted with 10 mg of the peptide comprised of the amino acid sequence of formula (II) "Cys Lys Pro Asp Ala Ala Lys Lys Gly Val Val Lys Ala Glu Lys Ser" synthesized in Example 2, according to the instruction manual. Thus, an affinity chromatography column was prepared in which the above synthetic peptide had been immobilized.
(ii) The column was equilibrated in advance with PBS. Subsequently, the globulin fractions obtained in (7) above were run through the column.
(iii) The column was thoroughly washed with PBS and then a 0.1M acetate buffer (pH3.0) was run through the column.
(iv) Elution fractions were collected, dialyzed against PBS, and concentrated.

Antibodies (polyclonal antibodies) that bind to the peptide comprised of the amino acid sequence of formula (II) "Cys Lys Pro Asp Ala Ala Lys Lys Gly Val Val Lys Ala Glu Lys Ser" were obtained by the above procedures of affinity chromatography from the 10 immunized rabbits.
(9) Each antibody (polyclonal antibody) obtained in (8) above from the 10 rabbits was subjected to confirmation of its binding capability to human HMGB1; that is, antibody titer.
Antibody titers were measured by ELISA as described in (i) to (vii) below.
(i) Human HMGB1 obtained in Example 4 was dissolved in saline to a concentration of 1 µg/mL. Human HMGB1 was immobilized to the microplate by adding the solution in aliquots of 100 µL per well to a 96-well microplate (Nunc) and then let stand at 37°C for 2 hours.
(ii) Next, each well of the microplate was washed with the wash solution (PBS containing 0.05% Tween20, PBS's composition is described below).
Phosphate buffered saline (PBS): an aqueous solution of 5.59 mM disodium hydrogen phosphate, 1.47 mM potassium dihydrogen phosphate, 137 mM sodium chloride, and 2.68 mM potassium chloride, pH adjusted to 7.2).
Subsequently 10 mM potassium dihydrogen phosphate-dipotassium hydrogen phosphate buffer (pH 7.2) containing 1% BSA was added in aliquots of 300 µL per well and then let stand at 37°C for 2 hours for blocking. The wells were then washed again with the wash solution.
(iii) Next, the antibodies from the 10 rabbits obtained in (8) above were diluted to concentrations of 0.01 µg/mL, 0.1 µg/mL, and 1 µg/mL with PBS.
The diluted antibodies were each added in aliquots of 100 µL per well to the microplate of (ii) above and then let stand at 37°C for 2 hours for binding. Thereafter, the wells were washed with the wash solution.
(iv) Also, as a control, 100 µL each of PBS containing 1% BSA was added in the place of the above diluted solution of the antibodies and then let stand at 37°C for 2 hours. The wells were then washed with the wash solution.
(v) An anti-rabbit IgG antibody (Dako) labeled with POD was diluted by 5,000 fold with PBS containing 3% BSA. The solution was added in amounts of 100 µL per well to the microplate of
(iii) above and that of (iv) above and then let stand at 37°C for 2 hours for binding.
(vi) Next, each well of the microplate was washed with the wash solution. POD reaction solution was added in amounts of 100 µL per well, followed by reaction at room temperature. POD reaction solution was prepared by adding 2 µL of 1.7% hydrogen peroxide to 1 mL of a 50 mM disodium hydrogen phosphate-24 mM citrate buffer containing 3 mM 3,3',5,5'-tetramethylbenzidine [TMBZ], immediately before use.
Subsequently, 15 minutes later, 6N sulfuric acid was added in amounts of 150 µL per well to stop the reaction.
The absorbance of each well was measured at 450 nm using an EIA plate reader (Bio-Rad Laboratories Inc.).
(vii) Table 2 shows the results of measuring the absorbance of each antibody obtained from a total of 10 rabbits.

| Antibody concentration (µg/mL) | Rabbit 1 | Rabbit 2 | Rabbit 3 | Rabbit 4 | Rabbit 5 | Rabbit 6 | Rabbit 7 | Rabbit 8 | Rabbit 9 | Rabbit 10 |
|---|---|---|---|---|---|---|---|---|---|---|
| 0.000 | 0.000 | 0.000 | 0.000 | 0.000 | 0.000 | 0.000 | 0.000 | 0.000 | 0.000 | 0.000 |
| 0.010 | 0.002 | 0.002 | 0.004 | 0.001 | 0.001 | 0.070 | 0.001 | 0.001 | 0.001 | 0.001 |
| 0.100 | 0.130 | 0.093 | 0.090 | 0.088 | 0.090 | 0.450 | 0.001 | 0.020 | 0.015 | 0.010 |
| 1.000 | 0.540 | 0.643 | 0.870 | 0.734 | 0.911 | 1.952 | 0.320 | 0.450 | 0.330 | 0.350 |

| | | | | | | | | | | |
|---|---|---|---|---|---|---|---|---|---|---|
| [Measured value is absorbance at 450 nm] | | | | | | | | | | |

As shown in Table 2, when the concentration of an antibody 1 µg/mL, for example, an antibody from one rabbit out of 10 rabbits was found to have absorbance of 1.952. However, antibodies from the other 9 rabbits were found to have absorbance of 0.32 to 0.911, suggesting these 9 antibodies had low binding capability to human HMGB1.

Specifically, in the case of anti-human HMGB1 antibodies obtained by immunizing rabbits, the mammals, with the immunogen, the antibodies of 9 out of a total of 10 immunized rabbits were low-titer antibodies having low binding capability to human HMGB1.

Accordingly, it was confirmed that the probability of obtaining high-titer antibodies by immunizing rabbits with the immunogen is extremely low.

### [Example 6] (Confirmation of the reactivity of the avian-derived anti-human HMGB1 antibody to human HMGB1 and human HMGB2)

The reactivity of the avian-derived anti-human HMGB1 antibody prepared in Example 5 to human HMGB1 and human HMGB2 was confirmed by the Western blot method.
(1) Human HMGB1 (1 mg/mL) and HMGB2 (1 mg/mL) prepared in Example 4 were mixed in equivalent amounts. The solution was further mixed with a 125 mM tris(hydroxymethyl)aminomethane buffer (pH 6.8) containing 4% (w/v) sodium dodecyl sulfate, 20% (w/v) glycerin, and an appropriate amount of bromphenol blue in equivalent amounts.
(2) Next, the solution was subjected to electrophoresis using 15% SDS-polyacrylamide gel.
   Electrophoresis was done by applying a current of 20 mA for 180 minutes using barbital buffer (pH 8.8) as an electrophoresis buffer.
(3) Transfer following the electrophoresis of (2) above was done in a dry mode according to the instruction manual using a Nova-blot electrophoretic transfer kit (Pharmacia-LKB).
   First, the electrophoresis gel in (2) above was placed on a transfer device.
   Next, a 9 cm x 9 cm nitrocellulose membrane (Bio-Rad Laboratories Inc.) was laid over the gel. Then transfer was done using a transfer buffer comprising 48 mM tris (hydroxymethyl)aminomethane, 39 mM glycine, 0.0357% (W/V) sodium dodecyl sulfate (SDS), and 20% (V/V) methanol and at a current of 60 mA for 2 hours.
(4) The nitrocellulose membrane was immersed overnight at 4°C in 20 mL of PBS (aqueous solution (pH 7.2) containing 5.59 mM disodium hydrogen phosphate, 1.47 mM potassium dihydrogen phosphate, 137 mM sodium chloride, and 2.68 mM potassium chloride) containing 1% BSA for blocking.
   Next, the membrane was washed while shaking with 20 mL of the wash solution (PBS with 0.05% Tween20) for 10 minutes.
   The membrane was washed 3 times.
(5) Eighty (80) µg of the avian-derived anti-human HMGB1 antibody prepared in Example 5 was dissolved in 20 mL of PBS containing 1% BSA.
   The nitrocellulose membrane treated in (4) above was immersed in the solution at room temperature for 2 hours for reaction.
   Next, the membrane was washed while shaking with 20 mL of the wash solution (PBS with 0.05% Tween20) for 10 minutes.
   The membrane was washed 3 times.
(6) Next, an anti-chicken IgY antibody (Up-Data) labeled with POD was diluted by 500 fold with PBS containing 3% BSA to 20 mL. The nitrocellulose membrane of (5) above was immersed in the solution at room temperature for 2 hours for reaction.
   Subsequently, the nitrocellulose membrane was washed while shaking with 20 mL of the wash solution for 10 minutes.
   The membrane was washed for 3 times.
(7) Next, the nitrocellulose membrane of (6) above was immersed in 20 mL of PBS containing 0.025% 3,3'-diaminobenzidine tetrahydrochloride and 0.01% hydrogen peroxide for color development at room temperature for 15 minutes.
(8) Separately, a positive control was tested according to the procedures as described in (1) to (7) above, except for: the use of a "mouse-derived anti-human HMGB1·HMGB2 antibody (monoclonal antibody)" binding to both human HMGB1 and human HMGB2, instead of "the avian-derived anti-human HMGB1 antibody prepared in Example 5" in (5) above; and the use of an "anti-mouse IgG antibody labeled with POD" (Dako) instead of "the anti-chicken IgY antibody labeled with POD" in (6) above.
   The above mouse-derived anti-human HMGB1·HMGB2 antibody (monoclonal antibody) is produced by antibody-producing cells (hybridomas) previously prepared by the present inventors.
   The antibody-producing cell (hybridoma) was named MD78 and deposited on July 4, 2001, under FERM P-18405 with the International Patent Organism Depositary, National Institute of Advanced Industrial Science and Technology.
   Methods for preparing the above antibody-producing cells (hybridomas) prepared by the present inventors and the above mouse-derived anti-human HMGB1·HMGB2 antibody (monoclonal antibody) are as described in JP Patent Publication (Kokai) No. 2003-96099 (JP Patent Publication (Kokai) No. 2003-96099 A, described above).
(9) In a separate negative control test, the procedures of (1) to (4) above were also performed, then proceeded to step (6) above using a 1:1 mixture of an anti-chicken IgY antibody labeled with POD and an anti-mouse IgG antibody labeled with POD mixed, followed by step (7) above. The negative control used (contacted) neither the avian-derived anti-human HMGB1 antibody prepared in Example 5 above nor the mouse-derived anti-human HMGB1·HMGB2 antibody (monoclonal antibody).
(10) By the above procedures, Western blot result was obtained for the avian-derived anti-human HMGB1 antibody prepared in Example 5.

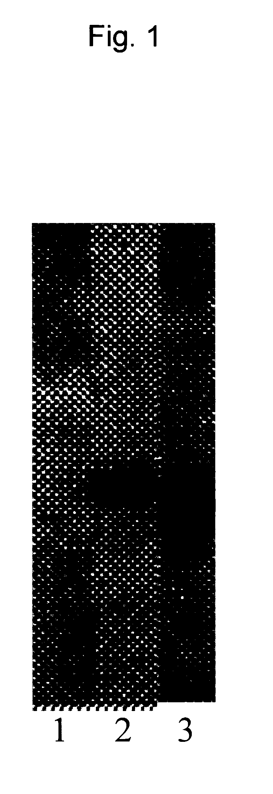

Fig. 1 shows the results of the Western blot.

In Fig. 1, "1" denotes the result of the negative control, "2" denotes the result of the avian-derived anti-human HMGB1 antibody prepared in Example 5, and "3" denotes the result of the positive control.

In Fig. 1, colored bands indicating human HMGB1 and human HMGB2 appeared in the lane "3" showing the result of the positive control of the mouse-derived anti-human HMGB1·HMGB2 antibody (monoclonal antibody) bound to both human HMGB1 and human HMGB2.

Of the two bands in "3," the upper colored band indicates human HMGB1 and the lower human HMGB2. This was confirmed from the band positions of electrophoresis separately performed for human HMGB1 and human HMGB2, followed by Western blotting.

Lane "2" shows the result obtained using the avian-derived anti-human HMGB1 antibody prepared in Example 5. A colored band is observed at the position to which human HMGB1 migrates; however, no color development can be observed at the position at which human HMGB2 is expected.

Thus, the results of the Western blot confirmed that the avian-derived anti-human HMGB1 antibody prepared in Example 5 reliably binds to human HMGB1, but not to human HMGB2.

Lane "1" shows the result of the negative control, in which neither the avian-derived anti-HMGB1 antibody prepared in Example 5 above nor the above mouse-derived anti-human HMGB1·HMGB2 antibody (monoclonal antibody) had been used (contacted). No color development was observed at positions where human HMGB1 or human HMGB2 are expected.

Therefore, it was confirmed that in each result for the Western blot method, non-specific color development had never taken place.

### [Example 7] (Preparation of the peroxidase-labeled anti-human HMGB1·HMGB2 antibody)

An antibody (monoclonal antibody) capable of binding to both human HMGB1 and human HMGB2 was labeled with peroxidase (POD), so that a peroxidase-labeled anti-human HMGB1·HMGB2 antibody was prepared.

### (1) Introduction of maleimide group into peroxidase

Four (4) mg of peroxidase (POD) [horseradish-derived] was dissolved in 0.3 mL of a 0.1 M phosphate buffer (pH 7.0). N-succinimidyl-4-(N-maleimidomethyl)cyclohexane-1-carboxylic acid (1.0 mg) dissolved in 60 µL of N,N'-dimethylformamide was added to the solution, followed by 60 minutes of reaction at 30°C.

Subsequently, dialysis was performed overnight with a 0.1 M phosphate buffer (pH 6.0).

A maleimide group was introduced into the above peroxidase by the above procedures.

### (2) Introduction of a thiol group into the anti-human HMGB1·HMGB2 antibody

S-acetyl mercapto succinic anhydride (0.6 mg) dissolved in 10 µL of N,N'-dimethylformamide was added to 0.5 mL of a 0.1 M phosphate buffer solution (pH 6.5) containing the mouse-derived anti-human HMGB1·HMGB2 antibody (monoclonal antibody) at a concentration of 10 mg/mL prepared by the present inventors as described in (8) of Example 6 above, followed by 30 minutes of reaction at room temperature.

Then, 20 µL of 0.1 M EDTA, 0.1 mL of a 0.1 M Tris hydrochloride buffer (pH 7.0), and 0.1 mL of 1 M hydroxylamine hydrochloride (pH 7.0) were each added to the solution and then left to stand at 30°C for 5 minutes.

Next the resultant was run through a Sephadex G-25 column equilibrated in advance with a 0.1 M phosphate buffer (pH 6.0) containing 5 mM EDTA. Simple gel filtration chromatography was performed, so that excessive S-acetyl mercapto succinic anhydride was removed and antibody fractions were collected.

A thiol group was introduced into the above mouse-derived anti-human HMGB1·HMGB2 antibody (monoclonal antibody) by the above procedures.

### (3) Preparation of peroxidase-labeled anti-human HMGB1·HMGB2 antibody

The peroxidase prepared in (1) above via introduction of a maleimide group and the mouse-derived anti-human HMGB1·HMGB2 antibody (monoclonal antibody) prepared in (2) above via introduction of a thiol group were mixed in equivalent amounts, followed by 20 hours of reaction at 30°C. Thus, peroxidase was introduced (for labeling) into the above antibodies.

The resultants were then run through an ultra gel AcA34 column equilibrated in advance with a 0.1 M phosphate buffer (pH 6.5), so that gel filtration chromatography was performed.

Each fraction of the gel filtration chromatography was subjected to 10% polyacrylamide electrophoresis for confirmation. Specifically, only the antibody fractions to which peroxidase had been bound were collected so as to avoid incorporation of unbound peroxidase.

The peroxidase-bound antibody fractions were concentrated, so that the mouse-derived anti-human HMGB1·HMGB2 antibody (monoclonal antibody) to which peroxidase had been bound; that is, the peroxidase-labeled anti-human HMGB1·HMGB2 antibody was obtained.

The protein concentration of a solution containing the peroxidase-labeled anti-human HMGB1·HMGB2 antibody was measured.

### [Example 8] (Microplate-immobilized anti-human HMGB1 antibody)

The avian-derived anti-human HMGB1 antibody (polyclonal antibody) prepared in Example 5 was immobilized on a microplate, so that the microplate-immobilized anti-human HMGB1 antibody was prepared.
(1) The avian-derived anti-HMGB1 antibody (polyclonal antibody) prepared in Example 5 was adjusted to a concentration of 5 µg/mL with phosphate buffered saline (5.59 mM disodium hydrogen phosphate, 1.47 mM potassium dihydrogen phosphate, 137 mM sodium chloride, and 2.68 mM potassium chloride (pH 7.2)). The solution was added in amounts of 100 µL per well to a 96-well microplate (Nunc) and then left to stand at 37°C for 2 hours. Thus, the antibody was caused to adsorb to each well of the microplate for immobilization.
(2) The microplate on which the antibody had been immobilized was washed with a wash (phosphate buffered saline containing 0.05% Tween20 (pH 7.2)). A 10 mM potassium dihydrogen phosphate-dipotassium hydrogen phosphate buffer (pH 7.2) containing 1% BSA was added in amounts of 300 µL per well. The wells were then left to stand at 37°C for 2 hours for blocking and then washed again with a wash.

The avian-derived anti-human HMGB1 antibody (polyclonal antibody) prepared in Example 5 was immobilized by the above procedures on the microplate, so that the microplate-immobilized anti-human HMGB1 antibody was prepared.

### [Example 9] (Immunoassay reagent and immunoassay method for human HMGB1 in a sample)

The human HMGB1 prepared in Example 4 was measured by enzyme immunoassay (sandwich method) using the peroxidase-labeled anti-human HMGB1·HMGB2 antibody prepared in Example 7 and the microplate-immobilized anti-human HMGB1 antibody prepared in Example 8 as immunoassay reagents.

Then a calibration curve in the immunoassay method was produced.

### 1. Measuring reagent

### (1) Peroxidase-labeled anti-human HMGB1·HMGB2 antibody

The peroxidase-labeled anti-human HMGB1·HMGB2 antibody prepared in Example 7 was used as an enzyme labeled antibody for the sandwich method in enzyme immunoassay.

### (2) Microplate-immobilized anti-human HMGB1 antibody

The microplate-immobilized anti-human HMGB1 antibody prepared in Example 8 was used as an immobilized antibody for the sandwich method in enzyme immunoassay.

### (3) Wash solution

Phosphate buffered saline (pH 7.2) containing 0.05% Tween20 was prepared and then used as a wash solution.

### (4) Peroxidase substrate solution

Two (2) µL of 1.7% hydrogen peroxide was added immediately before use to 1 mL of a 50 mM disodium hydrogen phosphate-24 mM citrate buffer containing 3 mM 3,3',5,5'-tetramethylbenzidine (TMBZ). The thus prepared solution was used as a substrate of peroxidase as a label; that is, a peroxidase substrate solution.

### (5) Stop solution

A 6 N sulfuric acid aqueous solution was prepared as a stop solution.

### 2. Sample

### (1) Sample containing human HMGB1

The solution containing human HMGB1 prepared in Example 4 was sufficiently dialyzed with a 50 mM potassium dihydrogen phosphate-dipotassium hydrogen phosphate buffer (pH 7.4) containing 0.01 % sodium azide.

After dialysis, the protein concentration of the solution containing human HMGB1 was measured by protein assay (Bio-Rad Laboratories Inc.).

The above solution containing human HMGB1 was diluted with a 50 mM potassium dihydrogen phosphate-dipotassium hydrogen phosphate buffer (pH 7.4) containing 0.01 % sodium azide, so that a sample containing human HMGB1 at a concentration of 80 ng/mL and a sample containing human HMGB1 at a concentration of 160 ng/mL were prepared.

### (2) 0 ng/mL sample

The above 50 mM potassium dihydrogen phosphate-dipotassium hydrogen phosphate buffer (pH 7.4) containing 0.01% sodium azide was used as a sample containing no human HMGB1 wherein the concentration of the human HMGB1 was 0 ng/mL.

### 3. Measurement by enzyme immunoassay (sandwich method)

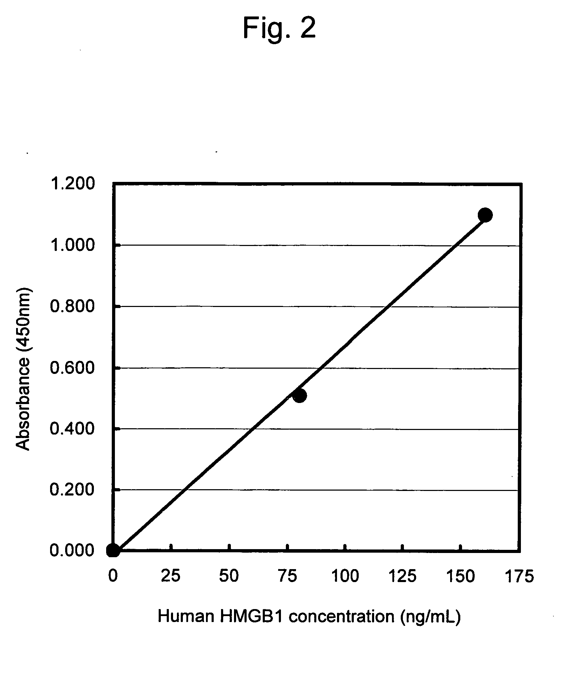

(1) The 2 types of sample prepared in 2 above, the sample containing human HMGB1 and the 0 ng/mL sample containing no human HMGB1 were each diluted 2 fold with normal saline.
(2) Each sample diluted in (1) above was added in amounts of 100 µL per well of the microplate-immobilized anti-human HMGB1 antibody in (2) of 1 above and then left to stand at 37°C for 12 hours, so that the antigen-antibody reaction of the antibody immobilized on the microplate with human HMGB1 contained in the sample was performed.
(3) Next, each well of the above microplate-immobilized anti-human HMGB1 antibody was washed with the wash solution in (3) of 1 above.
(4) The peroxidase-labeled anti-human HMGB1·HMGB2 antibody in (1) of 1 above was diluted 1,000 fold with phosphate buffered saline containing 3% BSA.
   Next, the solution was added in amounts of 100 µL per well (washed in (3) above) of the microplate-immobilized anti-human HMGB1 antibody and then left to stand at 37°C for 2 hours.
   Thus, a reaction was performed for binding the peroxidase-labeled anti-human HMGB1·HMGB2 antibody to human HMGB1 that had bound to the anti-human HMGB1 antibody immobilized on the microplate.
(5) Subsequently, each well of the microplate-immobilized anti-human HMGB1 antibody in (4) above was washed with the wash solution in (3) of 1 above.
(6) Next, the peroxidase substrate solution in (4) of 1 above was added in amounts of 100 µL per well of the microplate-immobilized anti-human HMGB1 antibody in (5) above.
   Then, the reaction was performed at room temperature.
(7) Fifteen (15) minutes after addition of the peroxidase substrate solution in (6) above, the stop solution in (5) of 1 above was added in amounts of 100 µL per well of the microplate-immobilized anti-human HMGB1 antibody, so as to stop the reaction of peroxidase as the label.
(8) Next, absorbance of the solution in each well of the microplate-immobilized anti-human HMGB1 antibody in (7) above was measured at 450 nm using a microplate reader (Bio-Rad Laboratories Inc.).
(9) Measured values of the above sample containing human HMGB1 and the sample containing no human HMGB1 (0 ng/mL), as obtained by the above procedures (that is, in the form of a calibration curve) are shown in Fig. 2.

In addition, in Fig. 2, the horizontal axis denotes the concentrations of human HMGB1 contained in the samples and the vertical axis denotes absorbance values measured at 450 nm.

Measured absorbance values shown herein were each obtained by subtracting the blank absorbance value of phosphate buffered saline (pH 7.2) containing 3% BSA.

### 4. Conclusion

As shown in Fig. 2, in the case of the above samples containing human HMGB1, the absorbance measured herein increased in proportion to the concentration of human HMGB1 contained. It was shown that measured values are obtained in proportion to the concentrations of human HMGB1 contained in a sample.

Therefore, it was confirmed that the concentration of human HMGB1 contained in a sample can be measured accurately and quantitatively by the immunoassay reagent and the immunoassay method for human HMGB1 in a sample of the present invention.

### [Example 10] (Measurement of samples containing serum using the immunoassay reagent and the immunoassay method for human HMGB1 of the present invention)

Accuracy of the immunoassay reagent and the immunoassay method for human HMGB1 in a sample of the present invention at the time of measurement of a sample containing serum was confirmed by measuring human HMGB1 contained in the sample containing serum by the regent and the method.

### 1. Measuring reagents

### "(1) Peroxidase-labeled anti-human HMGB1·HMGB2 antibody," "(2) Microplate-immobilized anti-human HMGB1 antibody," "(3) Wash solution," "(4) Peroxidase substrate solution," and "(5) Stop solution" of "1. Measuring reagent" in Example 9 were each used.

### 2. Samples

### (1) The solution containing human HMGB1 prepared in Example 4 was sufficiently dialyzed with a 50 mM potassium dihydrogen phosphate-dipotassium hydrogen phosphate buffer (pH 7.4) containing 0.01% sodium azide. After dialysis, the protein concentration of the solution containing human HMGB1 was measured by protein assay (Bio-Rad Laboratories Inc.).

The solution containing human HMGB1 was then diluted with a 50 mM potassium dihydrogen phosphate-dipotassium hydrogen phosphate buffer (pH 7.4) containing 0.01 % sodium azide, so that a solution containing human HMGB1 at a concentration of 80 ng/mL and a solution containing human HMGB1 at a concentration of 160 ng/mL were prepared.

### (2) The 2 types of solution prepared in (1) above were each diluted two fold with normal saline.

Then, a senun-free sample containing human HMGB1 at a concentration of 40 ng/mL and a serum-free sample containing human HMGB1 at a concentration of 80 ng/mL were prepared.

### (3) Furthermore, the 2 types of solution prepared in (1) above were each diluted 2 fold with human serum known to contain no human HMGB1 (the concentration of human HMGB1 was 0 ng/mL).

Subsequently, a serum-containing sample containing human HMGB1 at a concentration of 40 ng/mL and a serum-containing sample containing human HMGB1 at a concentration of 80 ng/mL were prepared.

### 3. Measurement by enzyme immunoassay (sandwich method)

(1) Each sample prepared in 2 above was added in amounts of 100 µL per well of the microplate-immobilized anti-human HMGB1 antibody in (2) of 1 above and then left to stand at 37°C for 12 hours. Thus, the antigen-antibody reaction of the antibody immobilized on the microplate with human HMGB1 contained in a sample was performed.
(2) Next, each well of the microplate-immobilized anti-human HMGB1 antibody in (1) above was washed with the wash solution in (3) of 1 above.
(3) The peroxidase-labeled anti-human HMGB1·HMGB2 antibody in (1) of 1 above was diluted 1,000 fold with phosphate buffered saline containing 3% BSA.
Next, the diluted solution was added in amounts of 100 µL per well (washed as in (2) above) of the microplate-immobilized anti-human HMGB1 antibody and then left to stand at 37°C for 2 hours.
Thus, the reaction was performed for binding the peroxidase-labeled anti-human HMGB1·HMGB2 antibody to human HMGB1 that had bound to the antibody immobilized on the microplate.
(4) Subsequently, each well of the microplate-immobilized anti-human HMGB1 antibody in (3) above was washed with the wash solution in (3) of 1 above.
(5) Next, the peroxidase substrate solution in (4) of 1 above was added in amounts of 100 µL per well of the microplate-immobilized anti-human HMGB1 antibody in (4) above.
The reaction was then performed at room temperature.
(6) Fifteen (15) minutes after addition of the peroxidase substrate solution in (5) above, the stop solution in (5) of 1 above was added in amounts of 100 µL per well of the microplate-immobilized anti-human HMBG1 antibody, so as to stop the reaction of peroxidase as the label.
(7) Next, absorbance of the solution in each well of the microplate-immobilized anti-human HMGB1 antibody in (6) above was measured at 450 nm using a microplate reader (Bio-Rad Laboratories Inc.).
(8) The above measured value (absorbance) of each sample and the percentages [B/A] (%) each obtained by dividing a measured value (absorbance) [B] of a serum-containing sample by a measured value (absorbance) [A] of a serum-free sample are shown in Table 3.
In addition, measured absorbance values shown herein were each obtained by subtracting the blank absorbance value of phosphate buffered saline (pH 7.2) containing 3% BSA.

| Human HMGB1 concentration (ng/mL) | Measured value (absorbance) of serum-free sample [A] | Measured value (absorbance) of serum-containing sample [B] | Percentage [B/A] (%) |
|---|---|---|---|
| 40 | 0.510 | 0.506 | 99 |
| 80 | 1.100 | 1.050 | 96 |

### 4. Conclusion

As shown in Table 3, the percentage [B/A] obtained by dividing the measured value (absorbance) [B] of a serum-containing sample by the measured value (absorbance) [A] of a serum-free sample was 99% in the case of the sample containing human HMGB1 at a concentration of 40 ng/mL and the percentage of the same was 96% in the case of the sample containing human HMGB1 at a concentration of 80 ng/mL.

That is, the measured value (absorbance) of a serum-containing sample was almost the same as that in the case of a serum-free sample.

Sera contain various ingredients, having complicated compositions (matrices). It was confirmed that the concentration of human HMGB1 in a sample containing such serum can be accurately measured by the immunoassay reagent and the immunoassay method for human HMGB1 in a sample of the present invention.

Therefore, it could be confirmed that the concentration of human HMGB1 contained in a biological sample such as serum can be accurately measured by the immunoassay reagent and the immunoassay method for human HMGB1 in a sample of the present invention.

## Claims

1. An avian-derived anti-human HMGB1 antibody, comprising an avian-derived antibody specifically binding to the amino acid sequence shown by the following formula (I): Lys Pro Asp Ala Ala Lys Lys Gly Val Val Lys Ala Glu Lys Ser (I) of human HMGB1.

2. The avian-derived anti-human HMGB1 antibody according to claim 1, which is obtained by immunizing birds with:
a peptide comprising the amino acid sequence shown by the following formula (I): Lys Pro Asp Ala Ala Lys Lys Gly Val Val Lys Ala Glu Lys Ser (I) of human HMGB1;
a peptide comprising an amino acid sequence obtained by subjecting the amino acid sequence shown by formula (I) to deletion, substitution, insertion, addition, or modification of one to several amino acid residues; or
a conjugate of the peptide and a carrier
as an immunogen.

3. The avian-derived anti-human HMGB1 antibody according to claim 1 or 2, wherein the avian-derived anti-human HMGB1 antibody has high capability of binding to human HMGB1 and the productivity thereof is high.

4. The avian-derived anti-human HMGB1 antibody according to any one of claims 1 to 3, wherein the birds are chickens.

5. An immunoassay method for measuring human HMGB1 in a sample using an antigen-antibody reaction with an antibody specifically binding to the human HMGB1, wherein an avian-derived anti-human HMGB1 antibody that is an avian-derived antibody specifically binding to the amino acid sequence shown by the following formula (I): Lys Pro Asp Ala Ala Lys Lys Gly Val Val Lys Ala Glu Lys Ser (I) of human HMGB1 is used.

6. The immunoassay method for human HMGB1 in a sample according to claim 5, wherein the avian-derived anti-human HMGB1 antibody is obtained by immunizing birds with:
a peptide comprising the amino acid sequence shown by the following formula (I): Lys Pro Asp Ala Ala Lys Lys Gly Val Val Lys Ala Glu Lys Ser (I) of human HMGB1;
a peptide comprising an amino acid sequence obtained by subjecting the amino acid sequence shown by formula (I) to deletion, substitution, insertion, addition, or modification of one to several amino acid residues; or
a conjugate of the peptide and a carrier
as an immunogen.

7. The immunoassay method for human HMGB1 in a sample according to claim 5 or 6, wherein the avian-derived anti-human HMGB1 antibody has high capability of binding to human HMGB1 and the productivity thereof is high.

8. The immunoassay method for human HMGB1 in a sample according to any one of claims 5 to 7, which is a highly sensitive immunoassay method capable of measuring human HMGB1 with high sensitivity.

9. The immunoassay method for human HMGB1 in a sample according to any one of claims 5 to 8, wherein the birds are chickens.

10. An immunoassay reagent for measuring human HMGB1 in a sample using an antigen-antibody reaction with an antibody specifically binding to the human HMGB1, containing an avian-derived anti-human HMGB1 antibody that is an avian-derived antibody specifically binding to the amino acid sequence shown by the following formula (I): Lys Pro Asp Ala Ala Lys Lys Gly Val Val Lys Ala Glu Lys Ser (1) of human HMGB1.

11. The immunoassay reagent for human HMGB1 in sample according to claim 10, wherein the avian-derived anti-human HMGB1 antibody is obtained by immunizing birds with:
a peptide comprising the amino acid sequence shown by the following formula (I): Lys Pro Asp Ala Ala Lys Lys Gly Val Val Lys Ala Glu Lys Ser (I) of human HMGB1;
a peptide comprising an amino acid sequence obtained by subjecting the amino acid sequence shown by formula (I) to deletion, substitution, insertion, addition, or modification of one to several amino acid residues; or
a conjugate of the peptide and a carrier
as an immunogen.

12. The immunoassay reagent for human HMGB1 in a sample according to claim 10 or 11, wherein the avian-derived anti-human HMGB1 antibody has high capability of binding to human HMGB1 and the productivity thereof is high.

13. The immunoassay reagent for human HMGB1 in a sample according to any one of claims 10 to 12, which is a highly sensitive immunoassay reagent capable of measuring human HMGB1 with high sensitivity.

14. The immunoassay reagent for human HMGB1 in a sample according to any one of claims 10 to 13, wherein the productivity of the immunoassay reagent is improved.

15. The immunoassay reagent for human HMGB1 in a sample according to any one of claims 10 to 14, wherein the birds are chickens.

16. A method for obtaining an avian-derived anti-human HMGB1 antibody, comprising immunizing birds with:
a peptide comprising the amino acid sequence shown by the following formula (I): Lys Pro Asp Ala Ala Lys Lys Gly Val Val Lys Ala Glu Lys Ser (I) of human HMGB1;
a peptide comprising an amino acid sequence obtained by subjecting the amino acid sequence shown by formula (I) to deletion, substitution, insertion, addition, or modification of one to several amino acid residues; or
a conjugate of the peptide and a carrier
as an immunogen.

17. The method for obtaining an avian-derived anti-human HMGB1 antibody according to claim 16, wherein the avian-derived anti-human HMGB1 antibody is capable of specifically binding to the amino acid sequence shown by the following formula (I): Lys Pro Asp Ala Ala Lys Lys Gly Val Val Lys Ala Glu Lys Ser (I) of human HMGB1.

18. The method for obtaining an avian-derived anti-human HMGB1 antibody according to claim 16 or 17, wherein the avian-derived anti-human HMGB1 antibody has high capability of binding to human HMGB1 and the productivity thereof is high.

19. The method for obtaining an avian-derived anti-human HMGB1 antibody according to any one of claims 16 to 18, wherein the birds are chickens.

20. A method for improving the productivity for obtaining an anti-human HMGB1 antibody, comprising immunizing birds with:
a peptide comprising the amino acid sequence shown by the following formula (I): Lys Pro Asp Ala Ala Lys Lys Gly Val Val Lys Ala Glu Lys Ser (I) of human HMGB1;
a peptide comprising an amino acid sequence obtained by subjecting the amino acid sequence
shown by formula (I) to deletion, substitution, insertion, addition, or modification of one to several amino acid residues; or
a conjugate of the peptide and a carrier
as an immunogen.

21. The method for improving the productivity for obtaining an anti-human HMGB1 antibody according to claim 20, wherein the anti-human HMGB1 antibody is capable of specifically binding to the amino acid sequence shown by the following formula (I): Lys Pro Asp Ala Ala Lys Lys Gly Val Val Lys Ala Glu Lys Ser (I) of human HMGB1.

22. The method for improving the productivity for obtaining an anti-human HMGB1 antibody according to claim 20 or 21, wherein the anti-human HMGB1 antibody has high capability of binding to human HMGB1.

23. The method for improving the productivity for obtaining an anti-human HMGB1 antibody according to any one of claims 20 to 22, wherein the birds are chickens.
